# EUROPEAN PATENT SPECIFICATION

(11) **EP 4 471 001 B1**
(45) Date of publication and mention of the grant of the patent: **12.11.2025**
(21) Application number: 24178389.3
(22) Date of filing: 28.05.2024
(51) Int. Cl.: C07C 1/32, C07C 17/16, C07C 29/10, C07C 41/22, C07C 41/26, C07C 41/30, C07C 67/14

(54) **HALOALKYL ALKOXYMETHYL ETHER COMPOUND, AND PROCESS FOR PREPARING 13,15-DIMETHYLHEPTACOSANE THEREFROM AND FOR PREPARING SYNTHETIC INTERMEDIATE THEREFOR**
HALOALKYLALKOXYMETHYLETHERVERBINDUNG UND VERFAHREN ZUR HERSTELLUNG VON 13,15-DIMETHYLHEPTACOSAN DARAUS UND ZUR HERSTELLUNG EINES SYNTHETISCHEN ZWISCHENPRODUKTS DAFÜR
COMPOSÉ HALOALKYL ALCOXYMÉTHYL ÉTHER ET PROCÉDÉ DE PRÉPARATION DE 13,15-DIMÉTHYLHEPTACOSANE À PARTIR DE CELUI-CI ET DE PRÉPARATION D'UN INTERMÉDIAIRE SYNTHÉTIQUE POUR CELUI-CI

(30) Priority: 31.05.2023 JP 2023090372
(43) Date of publication of application: 04.12.2024
(73) Proprietor: Shin-Etsu Chemical Co., Ltd., Tokyo 100-0005 (JP)
(72) Inventor: MIYAKE, Yuki, Niigata, 942-8601 (JP); WATANABE, Takeru, Niigata, 942-8601 (JP); KINSHO, Takeshi, Niigata, 942-8601 (JP); NAGAE, Yusuke, Niigata, 942-8601 (JP)
(74) Representative: De Vries & Metman

(56) References cited:
- "Major Hydrocarbons of the Post-Pharyngeal Glandsof Mated Queens of the Red Imported Fire AntSolenopsis invicta", LIPIDS, vol. 16, no. 7, 27 March 1981 (1981-03-27), pages 485 - 495, XP002812445

## Description

### TECHNICAL FIELD

The present invention relates to a haloalkyl alkoxymethyl ether compound, and also relates to a process for preparing 13,15-dimethylheptacosane from the haloalkyl alkoxymethyl ether compound and for preparing synthetic intermediate for preparing the haloalkyl alkoxymethyl ether compound.

### BACKGROUND ART

The Red imported fire ant (scientific name: *Solenopsis invicta*) is a sanitary pest originating in South America, and inflicts many pet and human bites each year in the United States and Taiwan. Bites of the Red imported fire ant are reported to be extremely painful (see Non-Patent Literature 1 below), and sometimes trigger allergic reactions, such as anaphylactic shock, which may cause death. Therefore, many countries are highly concerned about the invasion and establishment of the Red imported fire ant. In countries where the Red imported fire ant is already established, such as the United States, China, Taiwan, and Australia, efforts to employ pest control methods other than pesticides have been focused on the use of microsporidian protozoans (*Kneallhazia solenopsae*) or the entomopathogenic fungus *Beauveria bassiana,* as well as the use of cuticular hydrocarbons (see Non-Patent Literature 2 below).

Known cuticular hydrocarbons of the Red imported fire ant include heptacosane, 13-methylheptacosane, 13,15-dimethylheptacosane, 3-methylheptacosane, and 3,9-dimethylheptacosane (see Non-Patent Literature 1 below). The synthesis of 13,15-dimethylheptacosane, starting from 2-tetradecyl magnesium bromide and 2-methyltetradecenal is disclosed in the non-patent literature (Lipids, vol. 16, no. 7 (1981), Scheme II). The products of the Grignard reaction were treated with TsCl/Py and then lithium aluminium hydride reduction of the tosylate of alcohols gave 13,15-dimethylheptacosane.

### PRIOR ART

### [Non-Patent Literatures]

[Non-Patent Literature 1] Rong-Nan Huang et al., Int. J. Environ. Res. Public Health, 2021, 18, 5055.
[Non-Patent Literature 2] Robert K. Vander Meer et al., J. Chem. Ecol., 1989, 15 (7), 2115-2125.

### OBJECT OF THE INVENTION

As mentioned above, known cuticular hydrocarbons of the Red imported fire ant include heptacosane, 13-methylheptacosane, 13,15-dimethylheptacosane, 3-methylheptacosane, and 3,9-dimethylheptacosane. Although the disclosure focuses on 13,15-dimethylheptacosane among such cuticular hydrocarbons, the present invention is not limited thereto. While 13,15-dimethylheptacosane itself is a known compound, its preparation process is unknown.

The present invention has been made in view of the aforementioned circumstances, and aims to provide a novel synthetic intermediate useful for preparing 13,15-dimethylheptacosane, and a process for preparing the synthetic intermediate. The present invention also aims to provide an efficient, and preferably economic, process for preparing 13,15-dimethylheptacosane from the aforesaid synthetic intermediate.

### SUMMARY OF THE INVENTION

As a result of intensive research to overcome the aforesaid problems of the prior art, the present inventors found that a haloalkyl alkoxymethyl ether compound is a key intermediate that can comprehensively synthesize various compounds having a 1,3-dimethyl skeleton. The present inventors further found an inexpensive and efficient process for preparing the haloalkyl alkoxymethyl ether compound, and an efficient process for preparing 13,15-dimethylheptacosane, which is a cuticular hydrocarbon compound of the Red imported fire ant, from the haloalkyl alkoxymethyl ether compound, and thus have completed the present invention. The aforesaid preparation process also was found to be suited to industrial application.

According to a first aspect of the present invention, there is provided a process for preparing a haloalkyl alkoxymethyl ether compound of the following general formula (1B): 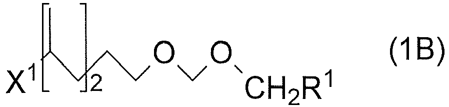
wherein X¹ represents a halogen atom, and R¹ represents a hydrogen atom, an n-alkyl group having 1 to 9 carbon atoms, or a phenyl group,
the process comprising the steps of
   converting a haloalkyl alkoxymethyl ether compound of the following general formula (1A): 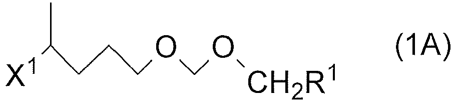
wherein X¹ and R¹ are as defined above,
into a nucleophilic reagent, 4-alkoxymethoxy-1-methylbutyl, of the following general formula (2A): 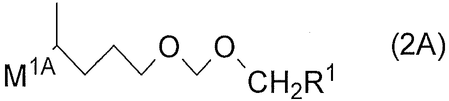
wherein M^{1A} represents Li, MgZ^{1A}, CuZ^{1A}, or CuLiZ^{1A}, Z^{1A} represents a halogen atom or a 4-alkoxymethoxy-1-methylbutyl group, and R¹ is as defined above,
subsequently subjecting the nucleophilic reagent, 4-alkoxymethoxy-1-methylbutyl (2A), to a nucleophilic addition reaction with propylene oxide of the following formula (3): 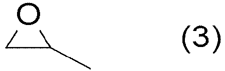
to obtain 6-hydroxy-4-methylheptyl alkoxymethyl ether compound of the following general formula (4): 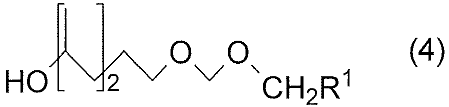
wherein R¹ is as defined above,
   and
   subjecting the 6-hydroxy-4-methylheptyl alkoxymethyl ether compound (4) to a halogenation reaction to form the aforesaid haloalkyl alkoxymethyl ether compound (1B).

According to a second aspect of the present invention, there is provided a process for preparing 4,6-dimethyloctadecanol of the following formula (7): 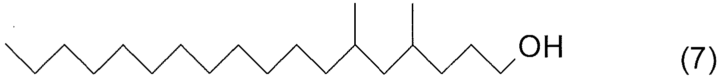 the process comprising the steps of
preparing a haloalkyl alkoxymethyl ether compound (1B), the process for preparing the haloalkyl alkoxymethyl ether compound (1B) being, for example, the aforesaid process for preparing the haloalkyl alkoxymethyl ether compound (1B) according to the first aspect, or another preparation process such as, for example, a process for preparing the haloalkyl alkoxymethyl ether compound (1B) according to any one of Examples 5 to 7 of the present specification,
converting the haloalkyl alkoxymethyl ether compound (1B) into a nucleophilic reagent, 6-alkoxymethoxy-1,3-dimethylhexyl, of the following general formula (2B): 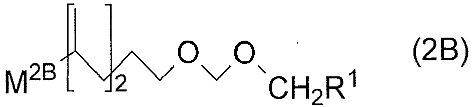 wherein M^{2B} represents Li, MgZ^{2B}, CuZ^{2B}, or CuLiZ^{2B}, Z^{2B} represents a halogen atom or a 6-alkoxymethoxy-1,3-dimethylhexyl group, and R¹ is as defined above;
subsequently subjecting the nucleophilic reagent, 6-alkoxymethoxy-1,3-dimethylhexyl (2B), to a coupling reaction with a 1-halododecane compound of the following general formula (5):

   X²(CH₂)₁₁CH₃ (5)

   wherein X² represents a halogen atom,
   to obtain a 4,6-dimethyloctadecyl alkoxymethyl ether compound of the following general formula (6): 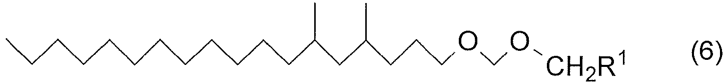
   wherein R¹ is as defined above,
      and
subjecting the 4,6-dimethyloctadecyl alkoxymethyl ether compound (6) to a dealkoxymethylation reaction to form the aforesaid 4,6-dimethyloctadecanol (7).

According to a third aspect of the present invention, there is provided a process for preparing 13,15-dimethylheptacosane of the following general formula (11): 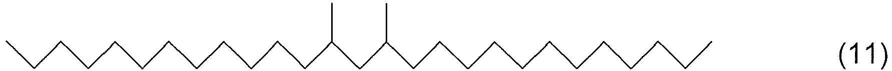 the process comprising the steps of
the aforesaid process for preparing 4,6-dimethyloctadecanol (7),
subjecting the 4,6-dimethyloctadecanol (7) to a halogenation reaction to obtain a 1-halo-4,6-dimethyloctadecane compound of the following general formula (8): 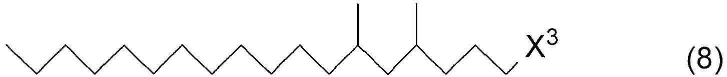 wherein X³ represents a halogen atom,
converting the 1-halo-4,6-dimethyloctadecane compound (8) into a nucleophilic reagent, 4,6-dimethyloctadecyl, of the following general formula (9): 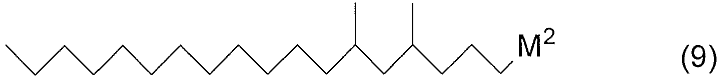 wherein M² represents Li, MgZ², CuZ², or CuLiZ², and Z² represents a halogen atom or a 4,6-dimethyloctadecyl group,
and subsequently subjecting the nucleophilic reagent, 4,6-dimethyloctadecyl (9), to a coupling reaction with a 1-halononane compound of the following general formula (10):

   X⁴(CH₂)₈CH₃ (10)

   wherein X⁴ represents a halogen atom,
   to form the aforesaid 13,15-dimethylheptacosane (11).

According to a fourth aspect of the present invention, there is provided a haloalkyl alkoxymethyl ether compound of the following general formula (1): 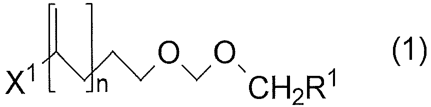 wherein X¹ represents a halogen atom, R¹ represents a hydrogen atom, an n-alkyl group having 1 to 9 carbon atoms, or a phenyl group, and n represents 1 or 2.

According to the present invention, it is possible to prepare a haloalkyl alkoxymethyl ether compound that is a building block for comprehensively synthesizing compounds having a 1,3-dimethyl skeleton. The haloalkyl alkoxymethyl ether compound can be used as a key intermediate to efficiently prepare 13,15-dimethylheptacosane, a compound having a 1,3-dimethyl skeleton, with less environmental impact and fewer steps. The preparation process is also economic.

### DETAILED DESCRIPTION OF THE INVENTION

### I. Haloalkyl alkoxymethyl ether compound of the following general formula (1)

First, the haloalkyl alkoxymethyl ether compound (1) will be explained in detail below.

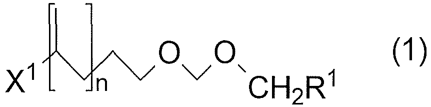

In the general formula (1) above, X¹ represents a halogen atom. Examples of the halogen atom X¹ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In the general formula (1) above, R¹ represents a hydrogen atom, an n-alkyl group having 1 to 9 carbon atoms, or a phenyl group.

Examples of the n-alkyl group R¹ include a methyl group, an ethyl group, a propyl group, a butyl group, a pentyl group, a hexyl group, a heptyl group, an octyl group, and a nonyl group.
*n* represents an integer of 1 or 2.

Specific examples of the haloalkyl alkoxymethyl ether compound (1) include the following compounds:
4-halopentyl alkoxymethyl ether compounds (1: *n* = 1) such as 4-chloropentyl methoxymethyl ether, 4-bromopentyl methoxymethyl ether, 4-iodopentyl methoxymethyl ether, 4-chloropentyl ethoxymethyl ether, 4-bromopentyl ethoxymethyl ether, 4-iodopentyl ethoxymethyl ether, 4-chloropentyl propyloxymethyl ether, 4-bromopentyl propyloxymethyl ether, 4-iodopentyl propyloxymethyl ether, 4-chloropentyl butyloxymethyl ether, 4-bromopentyl butyloxymethyl ether, 4-iodopentyl butyloxymethyl ether, 4-chloropentyl pentyloxymethyl ether, 4-bromopentyl pentyloxymethyl ether, 4-iodopentyl pentyloxymethyl ether, 4-chloropentyl hexyloxymethyl ether, 4-bromopentyl hexyloxymethyl ether, 4-iodopentyl hexyloxymethyl ether, 4-chloropentyl heptyloxymethyl ether, 4-bromopentyl heptyloxymethyl ether, 4-iodopentyl heptyloxymethyl ether, 4-chloropentyl octyloxymethyl ether, 4-bromopentyl octyloxymethyl ether, 4-iodopentyl octyloxymethyl ether, 4-chloropentyl nonyloxymethyl ether, 4-bromopentyl nonyloxymethyl ether, 4-iodopentyl nonyloxymethyl ether, 4-chloropentyl decyloxymethyl ether, 4-bromopentyl decyloxymethyl ether, 4-iodopentyl decyloxymethyl ether, 4-chloropentyl benzyloxymethyl ether, 4-bromopentyl benzyloxymethyl ether, and 4-iodopentyl benzyloxymethyl ether (hereinafter, also referred to as "the haloalkyl alkoxymethyl ether compound (1A)" or "the 4-halopentyl alkoxymethyl ether compound (1A)"); and
6-halo-4-methylheptyl alkoxymethyl ether compounds (1: *n* = 2) such as 6-chloro-4-methylheptyl methoxymethyl ether, 6-bromo-4-methylheptyl methoxymethyl ether, 6-iodo-4-methylheptyl methoxymethyl ether, 6-chloro-4-methylheptyl ethoxymethyl ether, 6-bromo-4-methylheptyl ethoxymethyl ether, 6-iodo-4-methylheptyl ethoxymethyl ether, 6-chloro-4-methylheptyl propyloxymethyl ether, 6-bromo-4-methylheptyl propyloxymethyl ether, 6-iodo-4-methylheptyl propyloxymethyl ether, 6-chloro-4-methylheptyl butyloxymethyl ether, 6-bromo-4-methylheptyl butyloxymethyl ether, 6-iodo-4-methylheptyl butyloxymethyl ether, 6-chloro-4-methylheptyl pentyloxymethyl ether, 6-bromo-4-methylheptyl pentyloxymethyl ether, 6-iodo-4-methylheptyl pentyloxymethyl ether, 6-chloro-4-methylheptyl hexyloxymethyl ether, 6-bromo-4-methylheptyl hexyloxymethyl ether, 6-iodo-4-methylheptyl hexyloxymethyl ether, 6-chloro-4-methylheptyl heptyloxymethyl ether, 6-bromo-4-methylheptyl heptyloxymethyl ether, 6-iodo-4-methylheptyl heptyloxymethyl ether, 6-chloro-4-methylheptyl octyloxymethyl ether, 6-bromo-4-methylheptyl octyloxymethyl ether, 6-iodo-4-methylheptyl octyloxymethyl ether, 6-chloro-4-methylheptyl nonyloxymethyl ether, 6-bromo-4-methylheptyl nonyloxymethyl ether, 6-iodo-4-methylheptyl nonyloxymethyl ether, 6-chloro-4-methylheptyl decyloxymethyl ether, 6-bromo-4-methylheptyl decyloxymethyl ether, 6-iodo-4-methylheptyl decyloxymethyl ether, 6-chloro-4-methylheptyl benzyloxymethyl ether, 6-bromo-4-methylheptyl benzyloxymethyl ether, and 6-iodo-4-methylheptyl benzyloxymethyl ether (hereinafter, also referred to as "haloalkyl alkoxymethyl ether compound (1B)" or "6-halo-4-methylheptyl alkoxymethyl ether compound (1B)").

### II. Process for preparing the aforesaid 6-halo-4-methylheptyl alkoxymethyl ether compound (1B: n = 2 for compound (1))

One target compound of the present invention, 6-halo-4-methylheptyl alkoxymethyl ether compound (1B), is prepared from the 4-halopentyl alkoxymethyl ether compound (1A) according to the preparation process of the following chemical reaction formula:

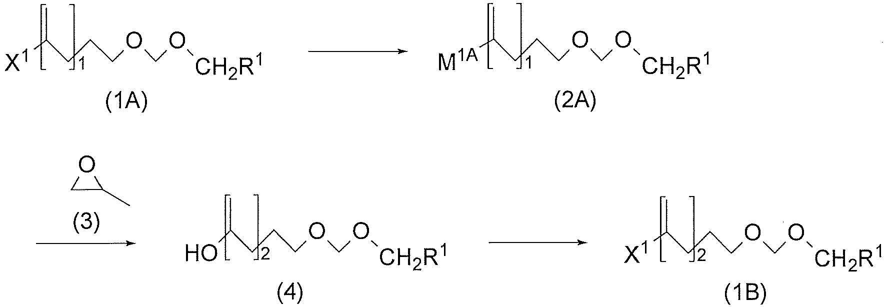

Namely, the 6-halo-4-methylheptyl alkoxymethyl ether compound (1B) may be prepared by converting the 4-halopentyl alkoxymethyl ether compound (1A) into the nucleophilic reagent, 4-alkoxymethoxy-1-methylbutyl (2A), then subjecting the nucleophilic reagent, 4-alkoxymethoxy-1-methylbutyl (2A), to a nucleophilic addition reaction with propylene oxide (3) to form the 6-hydroxy-4-methylheptyl alkoxymethyl ether compound (4), and then subjecting the 6-hydroxy-4-methylheptyl alkoxymethyl ether compound (4) to a halogenation reaction.

### (i) Nucleophilic reagent, 4-alkoxymethoxy-1-methylbutyl (2A), and preparation process thereof

### (a) The aforesaid nucleophilic reagent, 4-alkoxymethoxy-1-methylbutyl (2A), will be described in detail below.

The nucleophilic reagent, 4-alkoxymethoxy-1-methylbutyl (2A), is represented by the following general formula (2A):

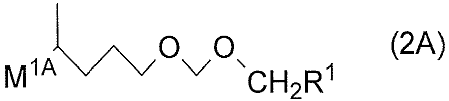

In the general formula (2A) above, M^{1A} represents Li, MgZ^{1A}, CuZ^{1A}, or CuLiZ^{1A}, and Z^{1A} represents a halogen atom or a 4-alkoxymethoxy-1-methylbutyl group. Examples of the halogen atom Z^{1A} include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In the general formula (2A) above, R¹ is as defined for the general formula (1), X¹ represents a halogen atom, and R¹ represents a hydrogen atom, an n-alkyl group having 1 to 9 carbon atoms, or a phenyl group.

When M^{1A} is MgZ^{1A} in the general formula (2A) above, the nucleophilic reagent, 4-alkoxymethoxy-1-methylbutyl (2A: M^{1A} = MgZ^{1A}), is a Grignard reagent.

Specific examples of the nucleophilic reagent, 4-alkoxymethoxy-1-methylbutyl (2A), include the following examples:
4-alkoxymethoxy-1-methylbutyllithium compounds (when M^{1A} = Li) such as 4-methoxymethoxy-1-methylbutyllithium, 4-ethoxymethoxy-1-methylbutyllithium, 4-propyloxymethoxy-1-methylbutyllithium, 4-butyloxymethoxy-1-methylbutyllithium, 4-pentyloxymethoxy-1-methylbutyllithium, 4-hexyloxymethoxy-1-methylbutyllithium, 4-heptyloxymethoxy-1-methylbutyllithium, 4-octyloxymethoxy-1-methylbutyllithium, 4-nonyloxymethoxy-1-methylbutyllithium, 4-decyloxymethoxy-1-methylbutyllithium, and 4-benzyloxymethoxy-1-methylbutyllithium;
4-alkoxymethoxy-1-methylbutylmagnesium halide compounds (when M^{1A} = MgZ^{1A}) such as 4-methoxymethoxy-1-methylbutylmagnesium chloride, 4-methoxymethoxy-1-methylbutylmagnesium bromide, 4-methoxymethoxy-1-methylbutylmagnesium iodide, 4-ethoxymethoxy-1-methylbutylmagnesium chloride, 4-ethoxymethoxy-1-methylbutylmagnesium bromide, 4-ethoxymethoxy-1-methylbutylmagnesium iodide, 4-propyloxymethoxy-1-methylbutylmagnesium chloride, 4-propyloxymethoxy-1-methylbutylmagnesium bromide, 4-propyloxymethoxy-1-methylbutylmagnesium iodide, 4-butyloxymethoxy-1-methylbutylmagnesium chloride, 4-butyloxymethoxy-1-methylbutylmagnesium bromide, 4-butyloxymethoxy-1-methylbutylmagnesium iodide, 4-pentyloxymethoxy-1-methylbutylmagnesium chloride, 4-pentyloxymethoxy-1-methylbutylmagnesium bromide, 4-pentyloxymethoxy-1-methylbutylmagnesium iodide, 4-hexyloxymethoxy-1-methylbutylmagnesium chloride, 4-hexyloxymethoxy-1-methylbutylmagnesium bromide, 4-hexyloxymethoxy-1-methylbutylmagnesium iodide, 4-heptyloxymethoxy-1-methylbutylmagnesium chloride, 4-heptyloxymethoxy-1-methylbutylmagnesium bromide, 4-heptyloxymethoxy-1-methylbutylmagnesium iodide, 4-octyloxymethoxy-1-methylbutylmagnesium chloride, 4-octyloxymethoxy-1-methylbutylmagnesium bromide, 4-octyloxymethoxy-1-methylbutylmagnesium iodide, 4-nonyloxymethoxy-1-methylbutylmagnesium chloride 4-nonyloxymethoxy-1-methylbutylmagnesium bromide, 4-nonyloxymethoxy-1-methylbutylmagnesium iodide, 4-decyloxymethoxy-1-methylbutylmagnesium chloride, 4-decyloxymethoxy-1-methylbutylmagnesium bromide, 4-decyloxymethoxy-1-methylbutylmagnesium iodide, 4-benzyloxymethoxy-1-methylbutylmagnesium chloride, 4-benzyloxymethoxy-1-methylbutylmagnesium bromide, and 4-benzyloxymethoxy-1-methylbutylmagnesium iodide (i.e., Grignard reagents);
bis[4-alkoxymethoxy-1-methylbutyl]cuprate compounds (when M^{1A} = CuZ^{1A}) such as bis[4-methoxymethoxy-1-methylbutyl]cuprate, bis[4-ethoxymethoxy-1-methylbutyl]cuprate, bis[4-propyloxymethoxy-1-methylbutyl]cuprate, bis[4-butyloxymethoxy-1-methylbutyl]cuprate, bis[4-pentyloxymethoxy-1-methylbutyl]cuprate, bis[4-hexyloxymethoxy-1-methylbutyl]cuprate, bis[4-heptyloxymethoxy-1-methylbutyl]cuprate, bis[4-octyloxymethoxy-1-methylbutyl]cuprate, bis[4-nonyloxymethoxy-1-methylbutyl]cuprate, bis[4-decyloxymethoxy-1-methylbutyl]cuprate, and bis[4-benzyloxymethoxy-1-methylbutyl]cuprate; and
Gilman reagents (when M^{1A} = CuLiZ^{1A}) such as lithium bis[4-methoxymethoxy-1-methylbutyl]cuprate, lithium bis[4-ethoxymethoxy-1-methylbutyl]cuprate, lithium bis[4-propyloxymethoxy-1-methylbutyl]cuprate, lithium bis[4-butyloxymethoxy-1-methylbutyl]cuprate, lithium bis[4-pentyloxymethoxy-1-methylbutyl]cuprate, lithium bis[4-hexyloxymethoxy-1-methylbutyl]cuprate, lithium bis[4-heptyloxymethoxy-1-methylbutyl]cuprate, lithium bis[4-octyloxymethoxy-1-methylbutyl]cuprate, lithium bis[4-nonyloxymethoxy-1-methylbutyl]cuprate, lithium bis[4-decyloxymethoxy-1-methylbutyl]cuprate, and lithium bis[4-benzyloxymethoxy-1-methylbutyl]cuprate (i.e., Gilman reagents).

Grignard reagents such as 4-alkoxymethoxy-1-methylbutylmagnesium halide compounds are preferred. By using said 4-alkoxymethoxy-1-methylbutylmagnesium halide compounds, a preferred reactivity may be ensured.

### (b) A process for preparing the aforesaid nucleophilic reagent, 4-alkoxymethoxy-1-methylbutyl (2A), will be described in detail below.

The nucleophilic reagent, 4-alkoxymethoxy-1-methylbutyl (2A), may be prepared from the aforesaid 4-halopentyl alkoxymethyl ether compound (1A). The process for preparing the 4-halopentyl alkoxymethyl ether compound (1A) is elaborated in Section III below.

A process for preparing the nucleophilic reagent, 4-alkoxymethoxy-1-methylbutyl (2A), when M^{1A} is MgZ^{1A}, that is, the 4-alkoxymethoxy-1-methylbutylmagnesium halide reagent (2A: M^{1A} = MgZ^{1A}) (which is a Grignard reagent), will be described in detail as an example.

The 4-alkoxymethoxy-1-methylbutylmagnesium halide reagent (2A: when M^{1A} = MgZ^{1A} and Z^{1A} = halogen atom) may be prepared, for example, by reacting the aforesaid 4-halopentyl alkoxymethyl ether compound (1A) with magnesium in a solvent, as shown in the following chemical reaction formula:

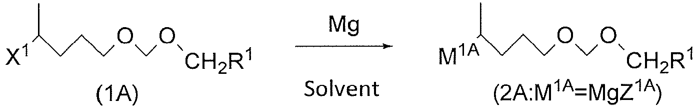

The amount of magnesium used, per mol of the 4-halopentyl alkoxymethyl ether compound (1A), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reaction rate of forming of the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming of the aforesaid Grignard reagent may be ensured.

The solvent may be used alone or in combination thereof, if necessary. The solvent may be a commercially available one.

The amount of the solvent used, per mol of the 4-halopentyl alkoxymethyl ether compound (1A), is preferably 30 to 5,000 g, and more preferably 50 to 3,000 g. By using said preferred amount and said more preferred amount, a preferred reactivity and a more preferred reactivity may be ensured.

The reaction temperature varies, depending on the solvent used, and is preferably 30 to 120°C. By using said preferred reaction temperature, a preferred reactivity may be ensured.

The reaction time varies, depending on the solvent used and/or production scale, and 0.5 to 100 hours is preferred. By using said reaction time, a preferred reactivity may be ensured.

### (ii) 6-Hydroxy-4-methylheptyl alkoxymethyl ether compound (4) and process for preparing 6-hydroxy-4-methylheptyl alkoxymethyl ether compound (4)

### (a) The aforesaid 6-hydroxy-4-methylheptyl alkoxymethyl ether compound (4) will be described in detail below.

The 6-hydroxy-4-methylheptyl alkoxymethyl ether compound (4) is represented by the following general formula (4):

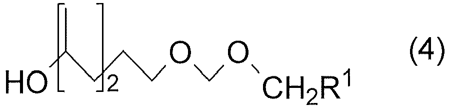

In the general formula (4) above, R¹ is as defined for the general formula (1).

Specific examples of the 6-hydroxy-4-methylheptyl alkoxymethyl ether compound (4) include 6-hydroxy-4-methylheptyl methoxymethyl ether, 6-hydroxy-4-methylheptyl ethoxymethyl ether, 6-hydroxy-4-methylheptyl propyloxymethyl ether, 6-hydroxy-4-methylheptyl butyloxymethyl ether, 6-hydroxy-4-methylheptyl pentyloxymethyl ether, 6-hydroxy-4-methylheptyl hexyloxymethyl ether, 6-hydroxy-4-methylheptyl heptyloxymethyl ether, 6-hydroxy-4-methylheptyl octyloxymethyl ether, 6-hydroxy-4-methylheptyl nonyloxymethyl ether, 6-hydroxy-4-methylheptyl decyloxymethyl ether, and 6-hydroxy-4-methylheptyl benzyloxymethyl ether.

### (b) A process for preparing the aforesaid 6-hydroxy-4-methylheptyl alkoxymethyl ether compound (4) will be described in detail below.

The 6-hydroxy-4-methylheptyl alkoxymethyl ether compound (4) may be prepared from the aforesaid nucleophilic reagent, 4-alkoxymethoxy-1-methylbutyl (2A), as shown in the following chemical reaction formula:

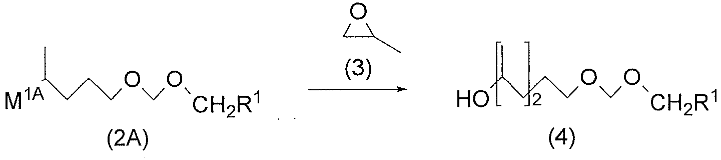

The preparation process includes at least a step of subjecting the nucleophilic reagent, 4-alkoxymethoxy-1-methylbutyl (2A), to a nucleophilic addition reaction with propylene oxide of the following general formula (3) to form the 6-hydroxy-4-methylheptyl alkoxymethyl ether compound (4).

In the aforesaid nucleophilic addition reaction, the nucleophilic reagent, 4-alkoxymethoxy-1-methylbutyl (2A), may be used alone or in combination thereof, if necessary.

Propylene oxide (3) may be a commercially available one, or may be synthesized in house.

The amount of the nucleophilic reagent, 4-alkoxymethoxy-1-methylbutyl (2A), per mol of propylene oxide (3), used in the nucleophilic addition reaction is preferably 0.6 to 1.3 mol. By using said preferred amount, preferred economy may be ensured.

A solvent may be incorporated in the nucleophilic addition reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), *N,N-*dimethylacetamide (DMAC), N-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the nucleophilic reagent, 4-alkoxymethoxy-1-methylbutyl (2A), is preferably 30 to 5,000 g, and more preferably 50 to 3,000 g. By using said preferred amount and said more preferred amount, a preferred reactivity and a more preferred reactivity may be ensured.

The nucleophilic addition reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred, and cuprous chloride is more preferred. By using said cuprous halides, a preferred reactivity may be ensured. By using said cuprous chloride, a more preferred reactivity may be ensured.

The catalyst may be used alone or in combination thereof, if necessary. The catalyst may be a commercially available one.

The amount of the catalyst used, per mol of the nucleophilic reagent, 4-alkoxymethoxy-1-methylbutyl (2A), is preferably 0.0001 to 0.300 mol, and more preferably 0.0003 to 0.100 mol. By using said preferred amount and said more preferred amount, a preferred reaction rate and/or post-treatment and a more preferred reaction rate and/or post-treatment may be ensured.

When the nucleophilic addition reaction is carried out in the presence of the catalyst, a co-catalyst may be used, if necessary. Examples of the co-catalyst include phosphorus compounds such as trialkyl phosphite compounds having 3 to 9 carbon atoms such as triethyl phosphite; and such as triarylphosphine compounds having 18 to 21 carbon atoms such as triphenylphosphine. A trialkyl phosphite compound having 3 to 9 carbon atoms, which is a liquid at a room temperature, is preferred. By using said trialkyl phosphite compound having 3 to 9 carbon atoms, a preferred handling may be ensured.

The co-catalyst may be used alone or in combination thereof, if necessary. The co-catalyst may be a commercially available one.

When the co-catalyst is used, the amount of the co-catalyst used, per mol of the nucleophilic reagent, 4-alkoxymethoxy-1-methylbutyl (2A), is preferably more than 0 up to 0.500 mol, and more preferably more than 0 up to 0.200 mol. By using said preferred amount and said more preferred amount, a preferred reactivity and a more preferred reactivity may be ensured.

When the nucleophilic addition reaction is carried out in the presence of the catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The lithium halide may be used alone or in combination thereof, if necessary. The lithium halide may be a commercially available one.

When the lithium halide is used, the amount of the lithium halide used, per mol of the nucleophilic reagent, 4-alkoxymethoxy-1-methylbutyl (2A), is preferably more than 0 up to 0.250 mol. By using said preferred amount, a preferred reactivity may be ensured.

The reaction temperature of the nucleophilic addition reaction varies, depending on the nucleophilic reagent, 4-alkoxymethoxy-1-methylbutyl (2A), used, and is preferably -78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the nucleophilic addition reaction varies, depending on the solvent used and/or production scale, and is preferably 0.5 to 100 hours. By using said preferred reaction time, a preferred reactivity may be ensured.

### (iii) 6-Halo-4-methylheptyl alkoxymethyl ether compound (1B) and process for preparing 6-halo-4-methylheptyl alkoxymethyl ether compound (1B)

(a) The 6-halo-4-methylheptyl alkoxymethyl ether compound (1B) is as mentioned above.
(b) A process for preparing the 6-halo-4-methylheptyl alkoxymethyl ether compound (1B) will be described in detail below.

The 6-halo-4-methylheptyl alkoxymethyl ether compound (1B) may be prepared from the aforesaid 6-hydroxy-4-methylheptyl alkoxymethyl ether compound (4).

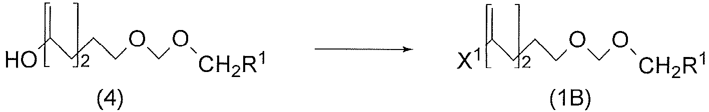

The preparation process includes a step of subjecting the 6-hydroxy-4-methylheptyl alkoxymethyl ether compound (4) to a halogenation reaction.

The halogenation reaction may be carried out by, for example, a process of tosylating a hydroxy group with a p-toluenesulfonyl halide compound and then halogenating with the metal salt, lithium halide compound; or a process of directly halogenating a hydroxy group with a halogenating agent.

Examples of the halogenating agent include halogens such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; p-toluenesulfonyl halide compounds such as p-toluenesulfonyl chloride, p-toluenesulfonyl bromide, and p-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, tert-butyldimethylsilyl chloride, tert-butyldimethylsilyl bromide, and tert-butyldimethylsilyl iodide; oxalyl halide compounds such as oxalyl chloride, oxalyl bromide, and oxalyl iodide; and N-halosuccinimide compounds such as N-chlorosuccinimide, N-bromosuccinimide, and N-iodosuccinimide. Methanesulfonyl halide compounds, benzenesulfonyl halide compounds, p-toluenesulfonyl halide compounds, and thionyl halide compounds are preferred. By using said methanesulfonyl halide compounds, benzenesulfonyl halide compounds, p-toluenesulfonyl halide compounds, and thionyl halide compounds, a preferred suppression of side reactions may be ensured. Methanesulfonyl halide compounds, benzenesulfonyl halide compounds, and thionyl halide compounds are particularly preferred. By using said methanesulfonyl halide compounds, benzenesulfonyl halide compounds, and thionyl halide compounds, a particularly preferred suppression of side reactions may be ensured.

The halogenating agent may be used alone or in combination thereof, if necessary. The halogenating agent may be a commercially available one.

The amount of the halogenating agent used, per mol of the 6-hydroxy-4-methylheptyl alkoxymethyl ether compound (4), is preferably 0.8 to 5.0 mol, and more preferably 1.0 to 2.5 mol. By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, N,N-diisopropylethylamine, piperidine, pyrrolidine, pyridine, lutidine, 4-dimethylaminopyridine, N,N-dimethylaniline, N,N-diethylaniline, and 1,8-diazabicyclo[5.4.0]-7-undecene (DBU).

When a methanesulfonyl halide compound, a benzenesulfonyl halide compound, a p-toluenesulfonyl halide compound, or the like is used as the aforesaid halogenating agent, the base is preferably amines, and more preferably pyridines such as pyridine, lutidine, and 4-dimethylaminopyridine.

When a thionyl halide compound is used as the halogenating agent, the base is preferably amines, and more preferably trialkylamines such as triethylamine.

The base may be used alone or in combination thereof, if necessary. The base may be a commercially available one.

When the base is used, the amount of the base used, per mol of the 6-hydroxy-4-methylheptyl alkoxymethyl ether compound (4), is preferably more than 0 up to 8.0 mol, and more preferably more than 0 up to 3.0 mol. By using said preferred amount and said more preferred amount, preferred yield and/or economy and a more preferred yield and/or economy may be ensured.

A metal salt may be added in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

When the halogenation with the metal salt, lithium halide compound, is carried out after the tosylation, the reaction is carried out with, for example, lithium salts such as lithium chloride, lithium bromide, and lithium iodide.

The metal salt may be used alone or in combination thereof, if necessary. The metal salt may be a commercially available one.

When the metal salt is used, the amount of the metal salt used, per mol of the 6-hydroxy-4-methylheptyl alkoxymethyl ether compound (4), is preferably more than 0 up to 30.0 mol, and more preferably more than 0 up to 5.0 mol. By using said preferred amount and said more preferred amount, a preferred reactivity and a more preferred reactivity may be ensured.

Although the metal salt increases the concentration of halide ions in the reaction system to thereby enhance the reactivity, it is preferred not to incorporate the metal salt in the reaction. By not incorporating the metal salt, preferred economy and/or environmental protection may be ensured.

A solvent may be incorporated in the halogenation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone, *N,N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. 2-Methyltetrahydrofuran, 4-methyltetrahydropyran, dichloromethane, chloroform, γ-butyrolactone, *N-methylpyrrolidone, N,N-dimethylformamide, N,N-dimethylacetamide,* and acetonitrile are preferred. By using said 2-methyltetrahydrofuran, 4-methyltetrahydropyran, dichloromethane, chloroform, γ-butyrolactone, *N-*methylpyrrolidone, *N,N-dimethylformamide, N,N*-dimethylacetamide, and acetonitrile, a preferred reactivity may be ensured. 2-Methyltetrahydrofuran, γ-butyrolactone, and acetonitrile are particularly preferred. By using said 2-methyltetrahydrofuran, γ-butyrolactone, and acetonitrile, particularly preferred safety may be ensured.

The solvent may be used alone or in combination thereof, if necessary. The solvent may be a commercially available one.

When a solvent is incorporated in the halogenation reaction, the amount of the solvent used, per mol of the 6-hydroxy-4-methylheptyl alkoxymethyl ether compound (4), is preferably more than 0 up to 3,000 g, and more preferably more than 0 up to 800 g.

The solvent may occupy part of the reactor space, which reduces the space for the starting materials, and reduces productivity. Therefore, the reaction may be carried out without a solvent, or with the base as the solvent.

The reaction temperature of the halogenation reaction varies, depending on the halogenating agent used, and is preferably 5 to 180°C, and more preferably 20 to 120°C. By using said preferred reaction temperature and said more preferred reaction temperature, a preferred reactivity and a more preferred reactivity may be ensured.

The reaction time of the halogenation reaction varies, depending on the halogenating agent used and/or production scale, and is preferably 0.5 to 100 hours. By using said preferred reaction time, a preferred reactivity may be ensured.

Thus, the 6-halo-4-methylheptyl alkoxymethyl ether compound (1B: *n* = 2 for compound (1)) may be prepared by three-carbon homologation from the haloalkyl alkoxymethyl ether compound, 4-halopentyl alkoxymethyl ether compound (1A: *n* = 1 for compound (1)). Then, the 6-halo-4-methylheptyl alkoxymethyl ether compound (1B) thus prepared may be used to prepare compounds such as 3,5-dimethyldodecanoic acid, 4,6-dimethyl-1-nonanamine, 4,6-dimethyl-1-undecanol, and 13,15-dimethylheptacosane having 1,3-dimethyl skeletons as in the following reaction formula:

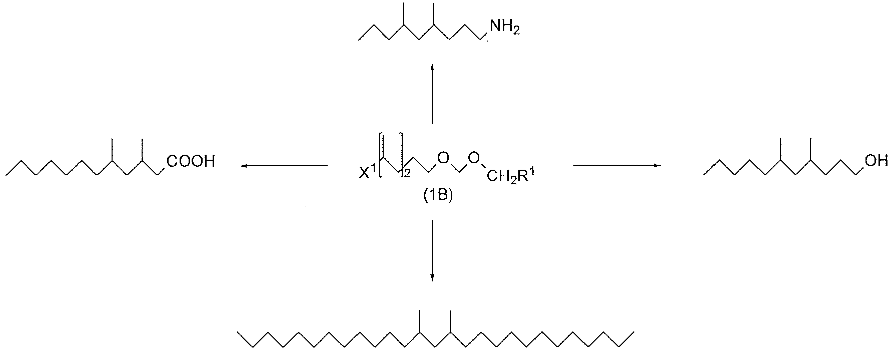

### III. Process for preparing the aforesaid haloalkyl alkoxymethyl ether compound (1A: n = 1 for compound (1))

One target compound of the present invention, 4-halopentyl alkoxymethyl ether compound (1A), may be prepared according to, for example, the preparation process of the following chemical reaction formula:

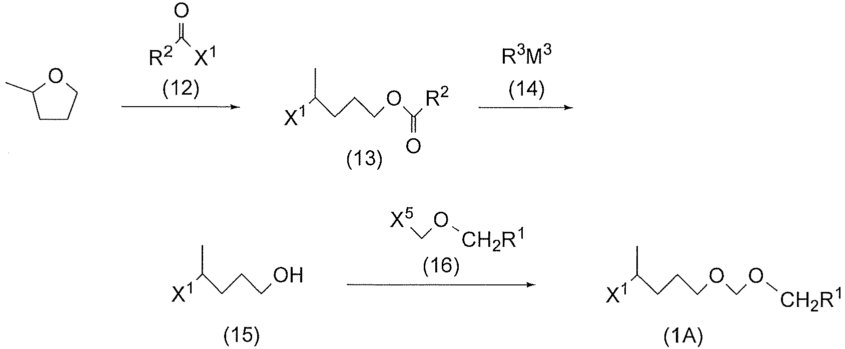

Namely, the 4-halopentyl alkoxymethyl ether compound (1A) may be prepared by opening the tetrahydrofuran ring of 2-methyltetrahydrofuran with an acid halide, followed by elimination of the acyl group, and then alkoxymethylation of the hydroxy group.

### (i) 4-Halopentyl acylate compound (13) and process for preparing 4-halopentyl acylate compound (13)

### (a) The 4-halopentyl acylate compound (13) will be described in detail below.

The 4-halopentyl acylate compound (13) is represented by the following general formula (13):

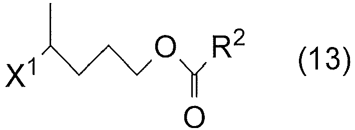

In the general formula (13) above, R² represents an alkyl group having 1 to 9 carbon atoms or a phenyl group, and X¹ is as defined for the general formula (1A).

Examples of the alkyl group R² include a methyl group, an ethyl group, a propyl group, a butyl group, a pentyl group, a hexyl group, a heptyl group, an octyl group, a nonyl group, and a tert-butyl group.

Examples of the halogen atom X¹ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

Specific examples of the 4-halopentyl acylate compound (13) include 4-chloropentyl acetate, 4-bromopentyl acetate, 4-iodopentyl acetate, 4-chloropentyl propionate, 4-bromopentyl propionate, 4-iodopentyl propionate, 4-chloropentyl butyrate, 4-bromopentyl butyrate, 4-iodopentyl butyrate, 4-chloropentyl valerate, 4-bromopentyl valerate, 4-iodopentyl valerate, 4-chloropentyl pivaloate, 4-bromopentyl pivaloate, 4-iodopentyl pivaloate, 4-chloropentyl benzoate, 4-bromopentyl benzoate, and 4-iodopentyl benzoate.

### (b) A process for preparing the aforesaid 4-halopentyl acylate compound (13) will be described in detail below.

The 4-halopentyl acylate compound (13) may be prepared using 2-methyltetrahydrofuran and the acid halide (12), as shown in the following chemical reaction formula:

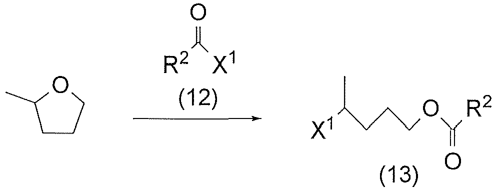

The preparation process includes a step of opening the tetrahydrofuran ring of 2-methyltetrahydrofuran with the acid halide (12).

The acid halide (12) will be described in detail below.

The acid halide (12) is represented by the following general formula (12):

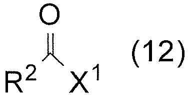

In the general formula (12), R² and X¹ are as defined for the general formula (13).

Specific examples of the acid halide (12) include acetyl halide compounds such as acetyl chloride, acetyl bromide, and acetyl iodide; propionyl halide compounds such as propionyl chloride, propionyl bromide, and propionyl iodide; butyryl halide compounds such as butyryl chloride, butyryl bromide, and butyryl iodide; valeryl halide compounds such as valeryl chloride, valeryl bromide, and valeryl iodide; pivaloyl halide compounds such as pivaloyl chloride, pivaloyl bromide, and pivaloyl iodide; and benzoyl halide compounds such as benzoyl chloride, benzoyl bromide, and benzoyl iodide.

The amount of the acid halide (12) used, per mol of 2-methyltetrahydrofuran, is preferably 0.7 to 1.5 mol, and more preferably 0.8 to 1.1 mol. By using said preferred amount and said more preferred amount, a preferred completion of the reaction and a more preferred completion of the reaction may be ensured.

The ring-opening reaction may be carried out in the presence of a catalyst, if necessary. Examples of the catalyst include zinc halide compounds such as zinc chloride, zinc bromide, and zinc iodide; titanium compounds such as titanium tetrachloride, titanium tetrabromide, titanium(IV) methoxide, titanium(IV) ethoxide, titanium(IV) isopropoxide, and titanium(IV) oxide; and zirconium compounds such as zirconium oxide. Zinc halide compounds are preferred. By using said zinc halide compounds, a preferred reactivity may be ensured.

The catalyst may be used alone or in combination thereof, if necessary. The catalyst may be a commercially available one.

The amount of the catalyst used, per mol of 2-methyltetrahydrofuran, is preferably 0.0001 to 0.3 mol, and more preferably 0.001 to 0.1 mol. By using said preferred amount and said more preferred amount, a preferred completion of the reaction and a more preferred completion of the reaction may be ensured.

A solvent may be incorporated in the ring-opening reaction, if necessary.

Examples of the solvent include those that do not affect the reaction such as, for example, hydrocarbon solvents such as hexane, heptane, benzene, toluene, xylene, and cumene. In view of easy preparation, 2-methyltetrahydrofuran may be used as both a solvent and a substrate without using another solvent.

The solvent may be used alone or in combination thereof, if necessary. The solvent may be a commercially available one.

When the solvent is used, the amount of the solvent used, per mol of 2-methyltetrahydrofuran, is preferably more than 0 up to 3,000 g, and more preferably more than 0 up to 500 g. By using said preferred amount and said more preferred amount, a preferred reactivity and a more preferred reactivity may be ensured.

The reaction temperature varies, depending on the production scale, and is preferably -15 to 85°C, and more preferably -10 to 45°C. By using said preferred reaction temperature and said more preferred reaction temperature, a preferred reactivity and a more preferred reactivity may be ensured.

The reaction time varies, depending on the solvent used and/or production scale, and is preferably 0.5 to 100 hours. By using said preferred reaction time, a preferred reactivity may be ensured.

### (ii) 4-Halo-1-pentanol compound (15) and process for preparing 4-halo-1-pentanol compound (15)

### (a) The 4-halo-1-pentanol compound (15) will be described in detail below.

The 4-halo-1-pentanol compound (15) is represented by the following general formula (15):

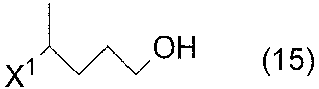

In the general formula (15) above, X¹ is as defined for the general formula (12).

Specific examples of the 4-halo-1-pentanol compound (15) include 4-chloro-1-pentanol, 4-bromo-1-pentanol, and 4-iodo-1-pentanol.

### (b) A process for preparing the aforesaid 4-halo-1-pentanol compound (15) will be described in detail below.

The 4-halo-1-pentanol compound (15) may be prepared by deacylating the acyl group of the aforesaid 4-halopentyl acylate compound (13) with the organometallic reagent R³M³ (14).

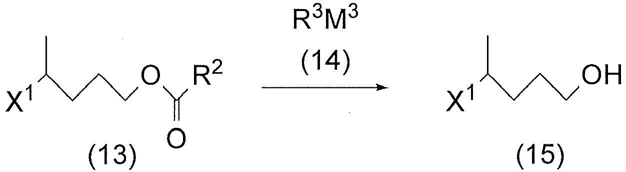

In the general formula (14) above, R³ represents an alkyl group having 1 to 14 carbon atoms, and preferably 1 to 8 carbon atoms, or an ethynyl group.

Examples of the alkyl group R³ include a methyl group, an ethyl group, a propyl group, a butyl group, a pentyl group, a hexyl group, a heptyl group, an octyl group, a nonyl group, a decyl group, an undecyl group, a dodecyl group, a tridecyl group, and a tetradecyl group.

In the general formula (14) above, M³ represents Li, Na, K, Ag, MgZ⁴, or CaZ⁴, and Z⁴ represents a halogen atom or R³. Examples of the halogen atom Z⁴ include a chlorine atom, a bromine atom, and an iodine atom.

Specific examples of the organometallic reagent R³M³ (14) include Grignard reagents such as alkyllithiums such as methyllithium, ethyllithium, propyllithium, n-butyllithium, sec-butyllithium, tert-butyllithium, pentyllithium, hexyllithium, heptyllithium, octyllithium, nonyllithium, and decyllithium; methylmagnesium halide compounds such as methylmagnesium chloride, methylmagnesium bromide, and methylmagnesium iodide; ethylmagnesium halide compounds such as ethylmagnesium chloride, ethylmagnesium bromide, and ethylmagnesium iodide; propylmagnesium halide compounds such as propylmagnesium chloride, propylmagnesium bromide, and propylmagnesium iodide; butylmagnesium halide compounds such as butylmagnesium chloride, butylmagnesium bromide, and butylmagnesium iodide; pentylmagnesium halide compounds such as pentylmagnesium chloride, pentylmagnesium bromide, and pentylmagnesium iodide; hexylmagnesium halide compounds such as hexylmagnesium chloride, hexylmagnesium bromide, and hexylmagnesium iodide; heptylmagnesium halide compounds such as heptylmagnesium chloride, heptylmagnesium bromide, and heptylmagnesium iodide; octylmagnesium halide compounds such as octylmagnesium chloride, octylmagnesium bromide, and octylmagnesium iodide; nonylmagnesium halide compounds such as nonylmagnesium chloride, nonylmagnesium bromide, and nonylmagnesium iodide; decylmagnesium halide compounds such as decylmagnesium chloride, decylmagnesium bromide, and decylmagnesium iodide; and metal acetylides such as lithium acetylide, sodium acetylide, potassium acetylide, calcium acetylide, and silver acetylide. Grignard reagents are preferred. By using said Grignard reagents, a preferred reactivity may be ensured.

The amount of the organometallic reagent R³M³ (14) used, per mol of the 4-halopentyl acylate compound (13), is preferably 1.5 to 5.0 mol, and more preferably 2.0 to 3.5 mol. By using said preferred amount and said more preferred amount, a preferred completion of the reaction and a more preferred completion of the reaction may be ensured.

A solvent may be incorporated in the deacylation, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), *N,N-*dimethylacetamide (DMAC), N-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

When the solvent is incorporated in the deacylation, the amount of the solvent used, per mol of the 4-halopentyl acylate compound (13), is preferably more than 0 up to 5,000 g, and more preferably more than 0 up to 3,000 g. By using said preferred amount and said more preferred amount, a preferred reactivity and a more preferred reactivity may be ensured.

The reaction temperature of the deacylation varies, depending on the production scale, and is preferably -15 to 90°C, and more preferably 10 to 50°C. By using said preferred reaction temperature and said more preferred reaction temperature, a preferred reactivity and a more preferred reactivity may be ensured.

The reaction time of the deacylation varies, depending on the solvent used and/or production scale, and is preferably 0.5 to 100 hours. By using said preferred reaction time, a preferred reactivity may be ensured.

### (iii) 4-Halopentyl alkoxymethyl ether compound (1A) and process for preparing 4-halopentyl alkoxymethyl ether compound (1A)

(a) The 4-halopentyl alkoxymethyl ether compound (1A) is as mentioned above.
(b) A process for preparing the 4-halopentyl alkoxymethyl ether compound (1A) will be described in detail below.

The 4-halopentyl alkoxymethyl ether compound (1A) may be prepared from the aforesaid 4-halo-1-pentanol compound (15).

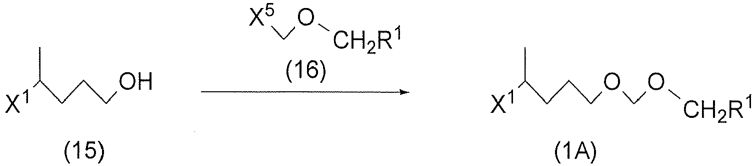

The preparation process includes a step of alkoxymethylating the 4-halo-1-pentanol compound (15) with the halomethyl alkyl ether compound (16).

The halomethyl alkyl ether compound (16) will be described in detail below.

The halomethyl alkyl ether compound (16) is represented by the following general formula (16):

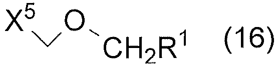

In the general formula (16) above, X⁵ represents a halogen atom. Examples of the halogen atom X⁵ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In the general formula (16) above, R¹ is as defined for the general formula (1A) above.

Specific examples of the halomethyl alkyl ether compound (16) include the following compounds:
chloromethyl alkyl ether compounds such as chloromethyl methyl ether, chloromethyl ethyl ether, chloromethyl propyl ether, chloromethyl butyl ether, chloromethyl pentyl ether, chloromethyl hexyl ether, chloromethyl heptyl ether, chloromethyl octyl ether, chloromethyl nonyl ether, and chloromethyl decyl ether;
chloromethyl benzyl ether;
bromomethyl alkyl ether compounds such as bromomethyl methyl ether, bromomethyl ethyl ether, bromomethyl propyl ether, bromomethyl butyl ether, bromomethyl pentyl ether, bromomethyl hexyl ether, bromomethyl heptyl ether, bromomethyl octyl ether, bromomethyl nonyl ether, and bromomethyl decyl ether;
bromomethyl benzyl ether;
iodomethyl alkyl ether compounds such as iodomethyl methyl ether, iodomethyl ethyl ether, iodomethyl propyl ether, iodomethyl butyl ether, iodomethyl pentyl ether, iodomethyl hexyl ether, iodomethyl heptyl ether, iodomethyl octyl ether, iodomethyl nonyl ether, and iodomethyl decyl ether; and
iodomethyl benzyl ether.

Chloromethyl methyl ether, chloromethyl ethyl ether, chloromethyl propyl ether, chloromethyl butyl ether, and chloromethyl benzyl ether are preferred. By using said chloromethyl methyl ether, chloromethyl ethyl ether, chloromethyl propyl ether, chloromethyl butyl ether, and chloromethyl benzyl ether, a preferred availability may be ensured. Chloromethyl methyl ether and chloromethyl ethyl ether are more preferred. By using said chloromethyl methyl ether and chloromethyl ethyl ether, a more preferred availability may be ensured.

The halomethyl alkyl ether compound (16) may be a commercially available one, or may be synthesized in house (see, for example, Example 3 below).

The amount of the halomethyl alkyl ether compound (16) used, per mol of the 4-halo-1-pentanol compound (15), is preferably 1.0 to 3.0 mol, and more preferably 1.0 to 1.8 mol. By using said preferred amount and said more preferred amount, a preferred completion of the reaction and a more preferred completion of the reaction may be ensured.

A base may be incorporated in the alkoxymethylation reaction, if necessary.

Examples of the base include amines such as triethylamine, *N,N-*diisopropylethylamine, piperidine, pyrrolidine, pyridine, lutidine, 4-dimethylaminopyridine, *N,N*-dimethylaniline, *N,N*-diethylaniline, *N,N*-dipropylaniline, N,N-dibutylaniline, and 1,8-diazabicyclo[5.4.0]-7-undecene (DBU).

The base may be used alone or in combination thereof, if necessary. The base may be a commercially available one.

The amount of the base used, per mol of the 4-halo-1-pentanol compound (15), is preferably 0.1 to 4.0 mol, and more preferably 1.0 to 2.5 mol. By using said preferred amount and said more preferred amount, a preferred yield and economy and a more preferred yield and economy may be ensured.

A solvent may be incorporated in the alkoxymethylation, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, *N,N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. Hydrocarbon solvents such as toluene and xylene; and ester solvents such as methyl acetate and ethyl acetate are preferred. By using said hydrocarbon solvents such as toluene and xylene; and ester solvents such as methyl acetate and ethyl acetate, a preferred reactivity may be ensured. The aforesaid alkoxymethylation may be carried out without a solvent to avoid reduced ease of preparation.

The solvent may be used alone or in combination thereof, if necessary. The solvent may be a commercially available one.

When a solvent is incorporated in the alkoxymethylation, the amount of the solvent used, per mol of the 4-halo-1-pentanol compound (15), is preferably more than 0 up to 1,500 g.

The reaction temperature of the alkoxymethylation varies, depending on the production scale, and is preferably -15 to 60°C, and more preferably 10 to 40°C. By using said preferred reaction temperature and said more preferred reaction temperature, a preferred reactivity and a more preferred reactivity may be ensured.

The reaction time of the alkoxymethylation varies, depending on the solvent used and/or production scale, and is preferably 0.5 to 100 hours. By using said preferred reaction time, a preferred reactivity may be ensured.

### IV. Process for preparing 13,15-dimethylheptacosane (11)

One target compound of the present invention, 13,15-dimethylheptacosane of the following formula (11), is prepared from the nucleophilic reagent, 6-alkoxymethoxy-1,3-dimethylhexyl, of the following general formula (2B) according to the preparation process of the following chemical reaction formula:

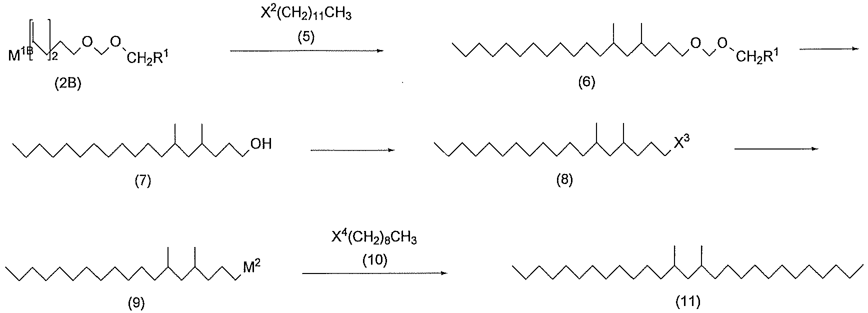

Namely, 13,15-dimethylheptacosane (11) may be prepared by subjecting the nucleophilic reagent, 6-alkoxymethoxy-1,3-dimethylhexyl (2B), to a coupling reaction with the 1-halododecane compound (5) to prepare the 4,6-dimethyloctadecyl alkoxymethyl ether compound (6), and then subjecting the 4,6-dimethyloctadecyl alkoxymethyl ether compound (6) to a dealkoxymethylation reaction to form 4,6-dimethyloctadecanol (7). The 4,6-dimethyloctadecanol (7) is then subjected to a halogenation reaction to form the 1-halo-4,6-dimethyloctadecane compound (8). The 1-halo-4,6-dimethyloctadecane compound (8) is then converted into the nucleophilic reagent, 4,6-dimethyloctadecyl (9), followed by subjecting the nucleophilic reagent, 4,6-dimethyloctadecyl (9), to a coupling reaction with the 1-halononane compound (10) to prepare 13,15-dimethylheptacosane (11).

### (i) 4,6-Dimethyloctadecyl alkoxymethyl ether compound (6) and process for preparing 4,6-dimethyloctadecyl alkoxymethyl ether compound (6)

### (a) The aforesaid 4,6-dimethyloctadecyl alkoxymethyl ether compound (6) will be described in detail below.

The 4,6-dimethyloctadecyl alkoxymethyl ether compound (6) is represented by the following general formula (6):

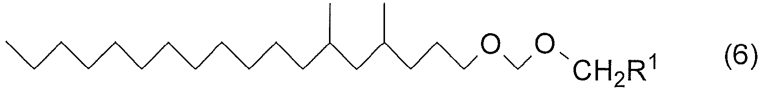

In the general formula (6) above, R¹ is as defined for the general formula (1A) above.

Specific examples of the 4,6-dimethyloctadecyl alkoxymethyl ether compound (6) include 4,6-dimethyloctadecyl methoxymethyl ether, 4,6-dimethyloctadecyl ethoxymethyl ether, 4,6-dimethyloctadecyl propyloxymethyl ether, 4,6-dimethyloctadecyl butyloxymethyl ether, 4,6-dimethyloctadecyl pentyloxymethyl ether, 4,6-dimethyloctadecyl hexyloxymethyl ether, 4,6-dimethyloctadecyl heptyloxymethyl ether, 4,6-dimethyloctadecyl octyloxymethyl ether, 4,6-dimethyloctadecyl nonyloxymethyl ether, 4,6-dimethyloctadecyl decyloxymethyl ether, and 4,6-dimethyloctadecyl benzyloxymethyl ether.

### (b) A process for preparing the aforesaid 4,6-dimethyloctadecyl alkoxymethyl ether compound (6) will be described in detail below.

The 4,6-dimethyloctadecyl alkoxymethyl ether compound (6) may be prepared from, for example, the nucleophilic reagent, 6-alkoxymethoxy-1,3-dimethylhexyl, of the following general formula (2B) and the 1-halododecane compound of the following general formula (5), as shown in the following chemical reaction formula:

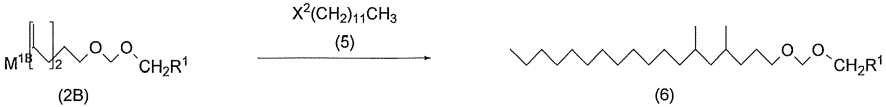

The preparation process includes a step of subjecting the nucleophilic reagent, 6-alkoxymethoxy-1,3-dimethylhexyl (2B), to a coupling reaction with the 1-halododecane compound (5).

The aforesaid nucleophilic reagent, 6-alkoxymethoxy-1,3-dimethylhexyl (2B), will be described in detail below.

The nucleophilic reagent, 6-alkoxymethoxy-1,3-dimethylhexyl (2B), is represented by the following general formula (2B):

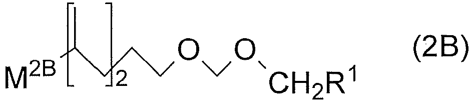

In the general formula (2B) above, M^{2B} represents Li, MgZ^{2B}, CuZ^{2B}, or CuLiZ^{2B}, and Z^{2B} represents a halogen atom or a 6-alkoxymethoxy-1,3-dimethylhexyl group. Examples of the halogen atom Z^{2B} include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

When M^{2B} is MgZ^{2B} in the general formula (2B) above, the nucleophilic reagent, 6-alkoxymethoxy-1,3-dimethylhexyl (2B: M^{2B} = MgZ^{2B}), is a Grignard reagent.

Specific examples of the nucleophilic reagent, 6-alkoxymethoxy-1,3-dimethylhexyl (2B), include the following:
6-alkoxymethoxy-1,3-dimethylhexyllithium compounds (when M^{2B} = Li) such as 6-methoxymethoxy-1,3-dimethylhexyllithium, 6-ethoxymethoxy-1,3-dimethylhexyllithium, 6-propyloxymethoxy-1,3-dimethylhexyllithium, 6-butyloxymethoxy-1,3-dimethylhexyllithium, 6-pentyloxymethoxy-1,3-dimethylhexyllithium, 6-hexyloxymethoxy-1,3-dimethylhexyllithium, 6-heptyloxymethoxy-1,3-dimethylhexyllithium, 6-octyloxymethoxy-1,3-dimethylhexyllithium, 6-nonyloxymethoxy-1,3-dimethylhexyllithium, 6-decyloxymethoxy-1,3-dimethylhexyllithium, and 6-benzyloxymethoxy-1,3-dimethylhexyllithium;
6-alkoxymethoxy-1,3-dimethylhexylmagnesium halide compounds (when M^{2B} = MgZ^{2B}) such as 6-methoxymethoxy-1,3-dimethylhexylmagnesium chloride, 6-methoxymethoxy-1,3-dimethylhexylmagnesium bromide, 6-methoxymethoxy-1,3-dimethylhexylmagnesium iodide, 6-ethoxymethoxy-1,3-dimethylhexylmagnesium chloride, 6-ethoxymethoxy-1,3-dimethylhexylmagnesium bromide, 6-ethoxymethoxy-1,3-dimethylhexylmagnesium iodide, 6-propyloxymethoxy-1,3-dimethylhexylmagnesium chloride, 6-propyloxymethoxy-1,3-dimethylhexylmagnesium bromide, 6-propyloxymethoxy-1,3-dimethylhexylmagnesium iodide, 6-butyloxymethoxy-1,3-dimethylhexylmagnesium chloride, 6-butyloxymethoxy-1,3-dimethylhexylmagnesium bromide, 6-butyloxymethoxy-1,3-dimethylhexylmagnesium iodide, 6-pentyloxymethoxy-1,3-dimethylhexylmagnesium chloride, 6-pentyloxymethoxy-1,3-dimethylhexylmagnesium bromide, 6-pentyloxymethoxy-1,3-dimethylhexylmagnesium iodide, 6-hexyloxymethoxy-1,3-dimethylhexylmagnesium chloride, 6-hexyloxymethoxy-1,3-dimethylhexylmagnesium bromide, 6-hexyloxymethoxy-1,3-dimethylhexylmagnesium iodide, 6-heptyloxymethoxy-1,3-dimethylhexylmagnesium chloride, 6-heptyloxymethoxy-1,3-dimethylhexylmagnesium bromide, 6-heptyloxymethoxy-1,3-dimethylhexylmagnesium iodide, 6-octyloxymethoxy-1,3-dimethylhexylmagnesium chloride, 6-octyloxymethoxy-1,3-dimethylhexylmagnesium bromide, 6-octyloxymethoxy-1,3-dimethylhexylmagnesium iodide, 6-nonyloxymethoxy-1,3-dimethylhexylmagnesium chloride, 6-nonyloxymethoxy-1,3-dimethylhexylmagnesium bromide, 6-nonyloxymethoxy-1,3-dimethylhexylmagnesium iodide, 6-decyloxymethoxy-1,3-dimethylhexylmagnesium chloride, 6-decyloxymethoxy-1,3-dimethylhexylmagnesium bromide, 6-decyloxymethoxy-1,3-dimethylhexylmagnesium iodide, 6-benzyloxymethoxy-1,3-dimethylhexylmagnesium chloride, 6-benzyloxymethoxy-1,3-dimethylhexylmagnesium bromide, and 6-benzyloxymethoxy-1,3-dimethylhexylmagnesium iodide (i.e., Grignard reagents);
bis[6-alkoxymethoxy-1,3-dimethylhexyl]cuprate compounds (when M^{2B} = CuZ^{2B}) such as bis[6-methoxymethoxy-1,3-dimethylhexyl]cuprate, bis[6-ethoxymethoxy-1,3-dimethylhexyl]cuprate, bis[6-propyloxymethoxy-1,3-dimethylhexyl]cuprate, bis[6-butyloxymethoxy-1,3-dimethylhexyl]cuprate, bis[6-pentyloxymethoxy-1,3-dimethylhexyl]cuprate, bis[6-hexyloxymethoxy-1,3-dimethylhexyl]cuprate, bis[6-heptyloxymethoxy-1,3-dimethylhexyl]cuprate, bis[6-octyloxymethoxy-1,3-dimethylhexyl]cuprate, bis[6-nonyloxymethoxy-1,3-dimethylhexyl]cuprate, bis[6-decyloxymethoxy-1,3-dimethylhexyl]cuprate, and bis[6-benzyloxymethoxy-1,3-dimethylhexyl]cuprate; and
lithium bis[6-alkoxymethoxy-1,3-dimethylhexyl]cuprate compounds (when M^{2B} = CuLiZ^{2B}) such as lithium bis[6-methoxymethoxy-1,3-dimethylhexyl]cuprate, lithium bis[6-ethoxymethoxy-1,3-dimethylhexyl]cuprate, lithium bis[6-propyloxymethoxy-1,3-dimethylhexyl]cuprate, lithium bis[6-butyloxymethoxy-1,3-dimethylhexyl]cuprate, lithium bis[6-pentyloxymethoxy-1,3-dimethylhexyl]cuprate, lithium bis[6-hexyloxymethoxy-1,3-dimethylhexyl]cuprate, lithium bis[6-heptyloxymethoxy-1,3-dimethylhexyl]cuprate, lithium bis[6-octyloxymethoxy-1,3-dimethylhexyl]cuprate, lithium bis[6-nonyloxymethoxy-1,3-dimethylhexyl]cuprate, lithium bis[6-decyloxymethoxy-1,3-dimethylhexyl]cuprate, and lithium bis[6-benzyloxymethoxy-1,3-dimethylhexyl]cuprate (i.e., Gilman reagents).
6-Alkoxymethoxy-1,3-dimethylhexylmagnesium halide compounds such as 6-methoxymethoxy-1,3-dimethylhexylmagnesium chloride, 6-methoxymethoxy-1,3-dimethylhexylmagnesium bromide, 6-methoxymethoxy-1,3-dimethylhexylmagnesium iodide, 6-ethoxymethoxy-1,3-dimethylhexylmagnesium chloride, 6-ethoxymethoxy-1,3-dimethylhexylmagnesium bromide, and 6-ethoxymethoxy-1,3-dimethylhexylmagnesium iodide, which are Grignard reagents, are preferred. By using said 6-alkoxymethoxy-1,3-dimethylhexylmagnesium halide compounds such as 6-methoxymethoxy-1,3-dimethylhexylmagnesium chloride, 6-methoxymethoxy-1,3-dimethylhexylmagnesium bromide, 6-methoxymethoxy-1,3-dimethylhexylmagnesium iodide, 6-ethoxymethoxy-1,3-dimethylhexylmagnesium chloride, 6-ethoxymethoxy-1,3-dimethylhexylmagnesium bromide, and 6-ethoxymethoxy-1,3-dimethylhexylmagnesium iodide, which are Grignard reagents, a preferred reactivity may be ensured.

The aforesaid 1-halododecane compound (5) will be described in detail below.

The 1-halododecane compound (5) is represented by the following general formula (5):

X²(CH₂)₁₁CH₃ (5)

In the general formula (5) above, X² represents a halogen atom. Examples of the halogen atom X² include a chlorine atom, a bromine atom, and an iodine atom. A bromine atom and an iodine atom are preferred. By using said bromine atom and iodine atom, a preferred reactivity may be ensured. A bromine atom is particularly preferred. By using said bromine atom, a particularly preferred reactivity may be ensured.

Specific examples of the 1-halododecane compound (5) include 1-chlorododecane, 1-bromododecane, and 1-iodododecane. 1-Bromododecane and 1-iodododecane are preferred. By using said 1-bromododecane and 1-iodododecane, a preferred yield may be ensured.

The 1-halododecane compound (5) may be used alone or in combination thereof, if necessary. The 1-halododecane compound (5) may be a commercially available one, or may be synthesized in house.

The 1-halododecane compound (5) may be prepared by, for example, subjecting 1-dodecanol to a halogenation reaction.

The coupling reaction of the nucleophilic reagent, 6-alkoxymethoxy-1,3-dimethylhexyl (2B), with the 1-halododecane compound (5) will be described in detail below.

The amount of the nucleophilic reagent, 6-alkoxymethoxy-1,3-dimethylhexyl (2B), per mol of the 1-halododecane compound (5), used in the coupling reaction is preferably 0.8 to 1.4 mol. By using said preferred amount, preferred economy may be ensured.

A solvent may be incorporated in the coupling reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the 1-halododecane compound (5), is preferably 30 to 5,000 g, and more preferably 50 to 3,000 g. By using said preferred amount and said more preferred amount, a preferred reactivity and a more preferred reactivity may be ensured.

The coupling reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of the 1-halododecane compound (5), is preferably 0.0003 to 0.3 mol, and more preferably 0.001 to 0.1 mol. By using said preferred amount and said more preferred amount, a preferred reaction rate and/or post-treatment and a more preferred reaction rate and/or post-treatment may be ensured.

When the coupling reaction is carried out in the presence of a catalyst, a co-catalyst may be used, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds are preferred. By using said trialkyl phosphite compounds, a preferred reactivity may be ensured.

The co-catalyst may be used alone or in combination thereof, if necessary. The co-catalyst may be a commercially available one.

The amount of the co-catalyst used, per mol of the 1-halododecane compound (5), is preferably 0.001 to 0.500 mol, and more preferably 0.005 to 0.200 mol. By using said preferred amount and said more preferred amount, a preferred reactivity and a more preferred reactivity may be ensured.

When the coupling reaction is carried out in the presence of a catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The lithium halide may be used alone or in combination thereof, if necessary. The lithium halide may be a commercially available one.

The amount of the lithium halide used in the coupling reaction, per mol of the 1-halododecane compound (5), is preferably 0.005 to 0.250 mol. By using said preferred amount, a preferred reactivity may be ensured.

The reaction temperature of the coupling reaction varies, depending on the nucleophilic reagent, 6-alkoxymethoxy-1,3-dimethylhexyl (2B), used, and is preferably - 78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the coupling reaction varies, depending on the solvent used and/or production scale, and is preferably 0.5 to 100 hours. By using said preferred reaction time, a preferred reactivity may be ensured.

### (ii) 4,6-Dimethyloctadecanol (7) and process for preparing 4,6-dimethyloctadecanol (7)

A process for preparing 4,6-dimethyloctadecanol (7) will be explained in detail below.

4,6-Dimethyloctadecanol (7) may be prepared, for example, from the aforesaid 4,6-dimethyloctadecyl alkoxymethyl ether compound (6), as shown in the following chemical reaction formula:

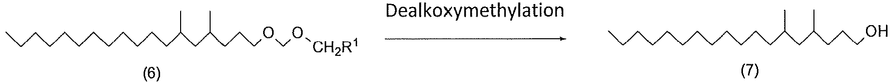

The preparation process includes a step of subjecting the 4,6-dimethyloctadecyl alkoxymethyl ether compound (6) to a dealkoxymethylation reaction.

Optimal conditions of the dealkoxymethylation reaction vary, depending on R¹. When R¹ is a phenyl group, the dealkoxymethylation can be carried out, for example, under Birch reduction conditions in which sodium is used in liquid ammonia. When R¹ is a methyl group, the dealkoxymethylation may be carried out using an acid, water, or an alcohol compound (17).

Examples of acid include inorganic acids such as hydrochloric acid and hydrobromic acid; sulfonic acids such as *p*-toluenesulfonic acid and benzenesulfonic acid; organic acids such as trifluoroacetic acid, acetic acid, formic acid, and oxalic acid; and Lewis acids such as iodotrimethylsilane and titanium tetrachloride. *P-*toluenesulfonic acid, benzenesulfonic acid, hydrochloric acid, and hydrobromic acid are preferred. By using said *p*-toluenesulfonic acid, benzenesulfonic acid, hydrochloric acid, and hydrobromic acid, a preferred suppression of side reactions may be ensured. *P-*toluenesulfonic acid, hydrochloric acid, and hydrobromic acid are particularly preferred. By using said *p*-toluenesulfonic acid, hydrochloric acid, and hydrobromic acid, a particularly preferred suppression of side reactions may be ensured.

The acid may be used alone or in combination thereof, if necessary. The acid may be a commercially available one.

The amount of the acid used, per mol of the 4,6-dimethyloctadecyl alkoxymethyl ether compound (6), is preferably 0.0001 to 10.0 mol, and more preferably 0.001 to 1.0 mol.

The alcohol compound (17) is represented by the following general formula:

R⁴OH (17)

In the general formula (17) above, R⁴ represents a monovalent hydrocarbon group having 1 to 15 carbon atoms, and preferably 1 to 6 carbon atoms. A monovalent hydrocarbon group having 1 to 6 carbon atoms is preferred. By using said monovalent hydrocarbon group having 1 to 6 carbon atoms, a preferred price or availability may be ensured. Examples of the monovalent hydrocarbon group include linear saturated hydrocarbon groups such as a methyl group, an ethyl group, an n-propyl group, an n-butyl group, an n-pentyl group, an n-hexyl group, an n-heptyl group, an *n*-octyl group, an n-nonyl group, an n-decyl group, an n-undecyl group, and an *n*-dodecyl group; branched saturated hydrocarbon groups such as an isopropyl group, a *sec*-butyl group, and a 2-methylbutyl group; linear unsaturated hydrocarbon groups such as a 2-propenyl group; branched unsaturated hydrocarbon groups such as a 2-methyl-2-propenyl group; cyclic saturated hydrocarbon groups such as a cyclopropyl group; and isomers thereof. A part of the hydrogen atoms in the hydrocarbon groups may be substituted with a methyl group, an ethyl group, or a hydroxy group.

The monovalent hydrocarbon group is preferably a methyl group, an ethyl group, an n-propyl group, and an n-butyl group. By using said methyl group, ethyl group, n-propyl group, and n-butyl group, a preferred ease of handling of the alcohol compound (17) (encompassing, for example, the price and ease of removability due to the low boiling point) and/or quantity of starting materials due to the alcohol compound use may be ensured.

Examples of the alcohol compound (17) include linear alcohols such as methanol, ethanol, n-propanol, n-butanol, n-pentanol, n-hexanol, n-heptanol, n-octanol, n-nonanol, n-decanol, n-undecanol, n-dodecanol, n-tridecanol, n-tetradecanol, and n-pentadecanol; branched alcohols such as isopropanol and 2-butanol; and diols such as ethylene glycol, propylene glycol, 2-methyl-1,3-propanediol, 2,2-dimethyl-1,3-propanediol, 1,2-dimethyl-1,3-propanediol, 1,3-dimethyl-1,3-propanediol, and 2-methyl-1,4-butanediol. Methanol and ethanol are preferred. By using said methanol and ethanol, a preferred reactivity may be ensured. Methanol is particularly preferred. By using said methanol, a particularly preferred reactivity may be ensured.

The alcohol compound (17) may be used alone or in combination thereof, if necessary. The alcohol compound (17) may be a commercially available one.

The amount of the alcohol compound (17) used, per mol of the 4,6-dimethyloctadecyl alkoxymethyl ether compound (6), is preferably 1 to 1,000 mol, and more preferably 1 to 100 mol. By using said preferred amount and said more preferred amount, a preferred reactivity and a more preferred reactivity may be ensured.

When water is used, the amount of the water used, per mol of the 4,6-dimethyloctadecyl alkoxymethyl ether compound (6), is preferably more than 0 up to 1,000 mol, and more preferably more than 0 up to 100 mol. By using said preferred amount and said more preferred amount, a preferred reactivity and a more preferred reactivity may be ensured.

A solvent other than the alcohol compound (17) may be incorporated in the dealkoxymethylation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N-*dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate.

The solvent may be used alone or in combination thereof, if necessary. The solvent may be a commercially available one.

When a solvent is incorporated in the dealkoxymethylation reaction, the amount of the solvent used, per mol of the 4,6-dimethyloctadecyl alkoxymethyl ether compound (6), is preferably more than 0 up to 2,000 g, and more preferably more than 0 up to 500 g.

The solvent may occupy part of the reactor space, which reduces the space for the starting materials, and reduces productivity. Therefore, the dealkoxymethylation may be carried out without a solvent.

The reaction temperature of the dealkoxymethylation varies, depending on the 4,6-dimethyloctadecyl alkoxymethyl ether compound (6) used, and is preferably -5 to 180°C, and more preferably 10 to 130°C. By using said preferred reaction temperature and said more preferred reaction temperature, a preferred reactivity and a more preferred reactivity may be ensured.

The reaction time of the dealkoxymethylation varies, depending on the 4,6-dimethyloctadecyl alkoxymethyl ether compound (6) used or production scale, and is preferably 0.5 to 100 hours. By using said preferred reaction time, a preferred reactivity may be ensured.

In the dealkoxymethylation, by-produced alkoxymethoxymethane may be distilled off from the reaction system, if necessary, whereby the equilibrium is shifted to the product side to reduce the reaction time.

### (iii) 1-Halo-4,6-dimethyloctadecane compound (8) and process for preparing 1-halo-4,6-dimethyloctadecane compound (8)

### (a) The aforesaid 1-halo-4,6-dimethyloctadecane compound (8) will be described in detail below.

The 1-halo-4,6-dimethyloctadecane compound (8) is represented by the following general formula (8):

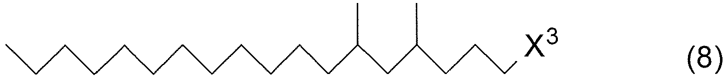

In the general formula (8) above, X³ represents a halogen atom. Examples of the halogen atom X³ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

### (b) A process for preparing the aforesaid 1-halo-4,6-dimethyloctadecane compound (8) will be described in detail below.

The 1-halo-4,6-dimethyloctadecane compound (8) may be prepared, for example, from 4,6-dimethyloctadecanol of the following general formula (7), as shown in the following chemical reaction formula:

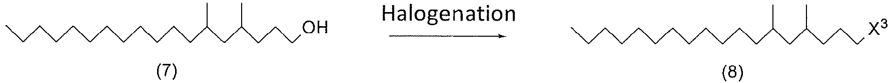

The preparation process includes a step of subjecting 4,6-dimethyloctadecanol (7) to a halogenation reaction.

The halogenation reaction may be carried out by, for example, a process of tosylating the hydroxy group with a *p*-toluenesulfonyl halide compound and then halogenating with the metal salt, lithium halide compound; or a process of directly halogenating the hydroxy group with a halogenating agent.

Examples of the halogenating agent include halogens such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; *p*-toluenesulfonyl halide compounds such as *p*-toluenesulfonyl chloride, *p*-toluenesulfonyl bromide, and *p*-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, *tert*-butyldimethylsilyl chloride, *tert*-butyldimethylsilyl bromide, and *tert*-butyldimethylsilyl iodide; oxalyl halide compounds such as oxalyl chloride, oxalyl bromide, and oxalyl iodide; and *N*-halosuccinimide compounds such as *N*-chlorosuccinimide, *N*-bromosuccinimide, and *N*-iodosuccinimide. Methanesulfonyl halide compounds, benzenesulfonyl halide compounds, *p*-toluenesulfonyl halide compounds, and thionyl halide compounds are preferred. By using said methanesulfonyl halide compounds, benzenesulfonyl halide compounds, *p*-toluenesulfonyl halide compounds, and thionyl halide compounds, a preferred suppression of side reactions may be ensured. Methanesulfonyl halide compounds, benzenesulfonyl halide compounds, and thionyl halide compounds are particularly preferred. By using said methanesulfonyl halide compounds, benzenesulfonyl halide compounds, and thionyl halide compounds, a particularly preferred suppression of side reactions may be ensured.

The halogenating agent may be used alone or in combination thereof, if necessary. The halogenating agent may be a commercially available one.

The amount of the halogenating agent used, per mol of 4,6-dimethyloctadecanol (7), is preferably 0.8 to 5.0 mol, and more preferably 1.0 to 2.5 mol. By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, *N*,*N*-diisopropylethylamine, piperidine, pyrrolidine, pyridine, lutidine, 4-dimethylaminopyridine, *N*,*N*-dimethylaniline, *N*,*N*-diethylaniline, and 1,8-diazabicyclo[5.4.0]-7-undecene (DBU).

When a methanesulfonyl halide compound, a benzenesulfonyl halide compound, a *p*-toluenesulfonyl halide compound, or the like is used as the halogenating agent, the base is preferably amines, and more preferably pyridines such as pyridine, lutidine, and 4-dimethylaminopyridine.

When a thionyl halide compound is used as the halogenating agent, the base is preferably amines, and more preferably trialkylamines such as triethylamine.

The base may be used alone or in combination thereof, if necessary. The base may be a commercially available one.

When the base is used, the amount of the base used, per mol of 4,6-dimethyloctadecanol (7), is preferably more than 0 up to 8.0 mol, and more preferably more than 0 up to 3.0 mol. By using said preferred amount and said more preferred amount, a preferred yield and/or economy and a more preferred yield and/or economy may be ensured.

A metal salt may be incorporated in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

When the halogenation with the metal salt, lithium halide compound, is carried out after the tosylation, the reaction is carried out with, for example, lithium salts such as lithium chloride, lithium bromide, and lithium iodide.

The metal salt may be used alone or in combination thereof, if necessary. The metal salt may be a commercially available one.

When the metal salt is used, the amount of the metal salt used, per mol of 4,6-dimethyloctadecanol (7), is preferably more than 0 up to 30.0 mol, and more preferably more than 0 up to 5.0 mol. By using said preferred amount and said more preferred amount, a preferred reactivity and a more preferred reactivity may be ensured.

Although the metal salt increases the concentration of halide ions in the reaction system to thereby enhance the reactivity, it is preferred not to incorporate the metal salt in the reaction. By not incorporating the metal salt, preferred economy and/or environmental protection may be ensured.

A solvent may be incorporated in the halogenation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N-*dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, acetone, *N*,*N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. 2-Methyltetrahydrofuran, 4-methyltetrahydropyran, dichloromethane, chloroform, γ-butyrolactone, *N*-methylpyrrolidone, *N*,*N*-dimethylformamide, *N*,*N*-dimethylacetamide, and acetonitrile are preferred. By using said 2-methyltetrahydrofuran, 4-methyltetrahydropyran, dichloromethane, chloroform, γ-butyrolactone, *N-*methylpyrrolidone, *N*,*N*-dimethylformamide, *N*,*N*-dimethylacetamide, and acetonitrile, a preferred reactivity may be ensured. 2-Methyltetrahydrofuran, γ-butyrolactone, and acetonitrile are particularly preferred. By using said 2-methyltetrahydrofuran, γ-butyrolactone, and acetonitrile, a particularly preferred safety may be ensured.

The solvent may be used alone or in combination thereof, if necessary. The solvent may be a commercially available one.

When a solvent is incorporated in the halogenation reaction, the amount of the solvent used, per mol of 4,6-dimethyloctadecanol (7), is preferably more than 0 up to 3,000 g, and more preferably more than 0 up to 800 g.

The solvent may occupy part of the reactor space, which reduces the space for the starting materials, and reduces productivity. Therefore, the reaction may be carried out without a solvent, or with the base as the solvent.

The reaction temperature of the halogenation reaction varies, depending on the halogenating agent used, and is preferably 5 to 180°C, and more preferably 20 to 120°C. By using said preferred reaction temperature and said more preferred reaction temperature, a preferred reactivity and a more preferred reactivity may be ensured.

The reaction time of the halogenation reaction varies, depending on the halogenating agent used and/or production scale, and is preferably 0.5 to 100 hours. By using said preferred reaction time, a preferred reactivity may be ensured.

### (iv) Nucleophilic reagent, 4,6-dimethyloctadecyl (9), and process for preparing nucleophilic reagent, 4,6-dimethyloctadecyl (9)

### (a) The nucleophilic reagent, 4,6-dimethyloctadecyl (9), will be described in detail below.

The nucleophilic reagent, 4,6-dimethyloctadecyl (9), is represented by the following general formula (9):

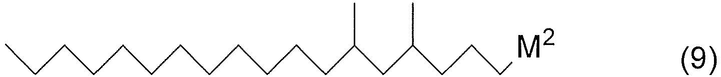

In the general formula (9) above, M² represents Li, MgZ², CuZ², or CuLiZ², and Z² represents a halogen atom or a 4,6-dimethyloctadecyl group. Examples of the halogen atom Z² include a chlorine atom, a bromine atom, and an iodine atom.

Specific examples of the nucleophilic reagent, 4,6-dimethyloctadecyl (9), include 4,6-dimethyloctadecyllithium (when M² = Li); 4,6-dimethyloctadecylmagnesium halide compounds (when M² = MgZ²) such as 4,6-dimethyloctadecylmagnesium chloride, 4,6-dimethyloctadecylmagnesium bromide, and 4,6-dimethyloctadecylmagnesium iodide (i.e., Grignard reagents); bis[4,6-dimethyloctadecyl]cuprate (when M² = CuZ²); and lithium bis[4,6-dimethyloctadecyl]cuprate (when M² = CuLiZ²) (i.e., Gilman reagents).

### (b) A process for preparing the aforesaid nucleophilic reagent, 4,6-dimethyloctadecyl (9), will be described in detail below.

The nucleophilic reagent, 4,6-dimethyloctadecyl (9), may be prepared in a conventional process or a process described below.

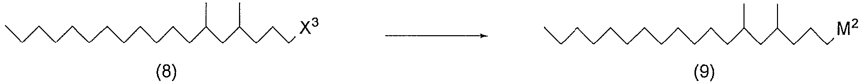

A process for preparing the nucleophilic reagent, 4,6-dimethyloctadecyl (9), in which M² is MgZ², that is, a 4,6-dimethyloctadecylmagnesium halide reagent (9: M² = MgZ²) (i.e., a Grignard reagent), will be described in detail below as an example.

The 4,6-dimethyloctadecylmagnesium halide reagent (9: M² = MgZ², Z² = halogen atom) may be prepared by, for example, reacting the aforesaid 1-halo-4,6-dimethyloctadecane compound (8) with magnesium in a solvent, as shown in the following chemical reaction formula:

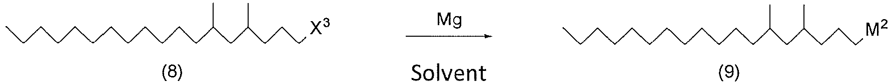

The amount of magnesium used, per mol of the 1-halo-4,6-dimethyloctadecane compound (8), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reaction rate of forming of the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming of the aforesaid Grignard reagent may be ensured.

The solvent may be used alone or in combination thereof, if necessary. The solvent may be a commercially available one.

The amount of the solvent used, per mol of the 1-halo-4,6-dimethyloctadecane compound (8), is preferably 30 to 5,000 g, and more preferably 50 to 3,000 g. By using said preferred amount and said more preferred amount, a preferred reactivity and a more preferred reactivity may be ensured.

The reaction temperature varies, depending on the solvent used, and is preferably 30 to 120°C. By using said preferred reaction temperature, a preferred reactivity may be ensured.

The reaction time varies, depending on the solvent used and/or production scale, and is preferably 0.5 to 100 hours. By using said preferred reaction time, a preferred reactivity may be ensured.

### (v) 13,15-Dimethylheptacosane (11) and process for preparing 13,15-dimethylheptacosane (11)

### (a) 13,15-Dimethylheptacosane (11) will be described in detail below.

13,15-Dimethylheptacosane (11) is represented by the following general formula (11):

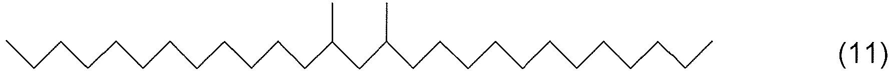

### (b) A process for preparing the aforesaid 13,15-dimethylheptacosane (11) will be described in detail below.

13,15-Dimethylheptacosane (11) may be prepared from, for example, the aforesaid nucleophilic reagent, 4,6-dimethyloctadecyl (9), and the 1-halononane compound of the following general formula (10), as shown in the following chemical reaction formula:

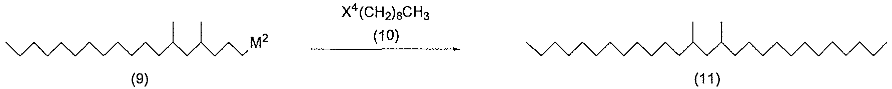

The preparation process includes a step of subjecting the nucleophilic reagent, 4,6-dimethyloctadecyl (9), to a coupling reaction with the 1-halononane compound (10).

The aforesaid 1-halononane compound (10) will be described in detail below.

The 1-halononane compound (10) is represented by the following general formula (10):

X⁴(CH₂)₈CH₃ (10)

In the general formula (10) above, X⁴ represents a halogen atom. Examples of the halogen atom X⁴ include a chlorine atom, a bromine atom, and an iodine atom. A bromine atom and an iodine atom are preferred. By using said bromine atom and iodine atom, a preferred reactivity may be ensured. A bromine atom is particularly preferred. By using said a bromine atom, a particularly preferred reactivity may be ensured.

Specific examples of the 1-halononane compound (10) include 1-chlorononane, 1-bromononane, and 1-iodononane. 1-Bromononane and 1-iodononane are preferred. By using said 1-bromononane and 1-iodononane, a preferred yield may be ensured.

The 1-halononane compound (10) may be used alone or in combination thereof, if necessary. The 1-halononane compound (10) may be a commercially available one, or may be synthesized in house.

The 1-halononane compound (10) may be prepared by, for example, subjecting nonanol to a halogenation reaction.

The coupling reaction of the nucleophilic reagent, 4,6-dimethyloctadecyl (9), with the 1-halononane compound (10) will be described in detail below.

The amount of the nucleophilic reagent, 4,6-dimethyloctadecyl (9), per mol of 1-halononane compound (10), used in the coupling reaction is preferably 0.8 to 1.4 mol. By using said preferred amount, preferred economy may be ensured.

A solvent may be incorporated in the coupling reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N-*dimethylformamide (DMF), *N,N-*dimethylacetamide (DMAC), *N*-methylpyrrolidone (NMP), dimethyl sulfoxide (DMSO), γ-butyrolactone(GBL), acetonitrile, *N*,*N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of 1-halononane compound (10), is preferably 30 to 5,000 g, and more preferably 50 to 3,000 g. By using said preferred amount and said more preferred amount, a preferred reactivity and a more preferred reactivity may be ensured.

The coupling reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of 1-halononane compound (10), is preferably 0.0003 to 0.3 mol, and more preferably 0.001 to 0.1 mol. By using said preferred amount and said more preferred amount, a preferred reaction rate and/or post-treatment and a more preferred reaction rate and/or post-treatment may be ensured.

When the coupling reaction is carried out in the presence of a catalyst, a co-catalyst may be used, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds are preferred. By using said trialkyl phosphite compounds, a preferred reactivity may be ensured.

The co-catalyst may be used alone or in combination thereof, if necessary. The co-catalyst may be a commercially available one.

The amount of the co-catalyst used, per mol of 1-halononane compound (10), is preferably 0.001 to 0.500 mol, and more preferably 0.005 to 0.200 mol. By using said preferred amount and said more preferred amount, a preferred reactivity and a more preferred reactivity may be ensured.

When the coupling reaction is carried out in the presence of a catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The lithium halide may be used alone or in combination thereof, if necessary. The lithium halide may be a commercially available one.

The amount of lithium halide used in the coupling reaction, per mol of 1-halononane compound (10), is preferably 0.005 to 0.250 mol. By using said preferred amount, a preferred reactivity may be ensured.

The reaction temperature of the coupling reaction varies, depending on the nucleophilic reagent, 4,6-dimethyloctadecyl (9), used, and is preferably -78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the coupling reaction varies, depending on the solvent used and/or production scale, and is preferably 0.5 to 100 hours. By using said preferred reaction time, a preferred reactivity may be ensured.

Thus, according to the present invention, the haloalkyl alkoxymethyl ether compound (1B) and the haloalkyl alkoxymethyl ether compound (1A), which are synthetic intermediates, can be easily and efficiently prepared. According to the present invention, the cuticular hydrocarbon of the Red imported fire ant (scientific name: *Solenopsis invicta*), 13,15-dimethylheptacosane (11), can be efficiently prepared with easy preparation and fewer steps, from the haloalkyl alkoxymethyl ether compound (1B) or from the haloalkyl alkoxymethyl ether compound (1A) via the haloalkyl alkoxymethyl ether compound (1B).

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 150°C, elevated in a rate of 5°C/min, and up to 230°C; column: DB-WAX, 0.25 µm x 0.25 mmϕ x 30 m; carrier gas: He (1.55 mL/min), detector: FID; column temperature: 150°C, elevated in a rate of 5°C/min, and up to 230°C.

The yield was calculated according to the following equation in consideration of purities (% GC) of a starting material and a product. Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, GBL represents γ-butyrolactone, PO represents propylene oxide, Ph represents a phenyl group, Bu represents a butyl group, and Et represents an ethyl group.

### Example 1: Preparation of 4-chloropentyl acetate (13: R² = CH₃, X¹ = Cl)

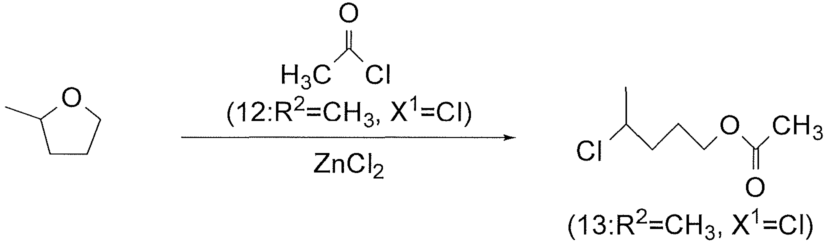

Zinc chloride (95.40 g, 0.70 mol) and 2-methyltetrahydrofuran (1,326.40 g, 15.40 mol) were placed in a reactor at a room temperature and stirred for 32 minutes at - 5 to 5°C. Acetyl chloride (12: R² = CH₃, X¹ = Cl) (1,099.00 g, 14.00 mol) was then added dropwise to the reactor at -5 to 5°C. After the completion of the dropwise addition, the reaction mixture was stirred for 2 hours at -5 to 5°C. Water (1,000 g) was then added to the reaction mixture, followed by phase separation. The organic layer thus obtained was subjected to distillation at a reduced pressure to obtain 4-chloropentyl acetate (13: R² = CH₃, X¹ = Cl) (2,051.43 g, 12.34 mol, purity 99.03%, b.p. = 71.0 to 78.9°C/0.40 kPa (3.0 mmHg)) with a yield of 88.11%.

The following is the spectrum data of 4-chloropentyl acetate (13: R² = CH₃, X¹ = Cl) thus obtained.

Nuclear magnetic resonance spectrum: ¹H-NMR (500 MHz, CDCl₃): δ = 1.51 (3H, J = 6.9 Hz), 1.67-1.91 (4H, m), 2.04 (3H, s), 3.99-4.12 (3H, m); ¹³C-NMR (500 MHz, CDCl₃): δ = 20.90, 25.32, 25.85, 36.65, 58.07, 63.77, 171.03.

Mass spectrum: EI-mass spectrum (70 eV): m/z 165 (M⁺+1), 121, 101, 86, 68, 43.

Infrared absorption spectrum: (D-ATR): v = 2971, 1739, 1446, 1365, 1240, 1036, 977, 875, 607.

### Example 2: Preparation of 4-chloro-1-pentanol (15: X¹ = Cl)

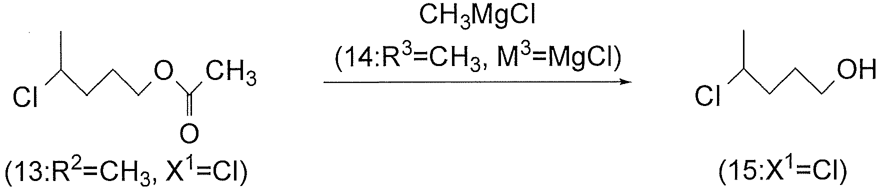

A THF solution (2,324.32 g) of methylmagnesium chloride (6.01 mol) was placed in a reactor at a room temperature and stirred for 12 minutes at 10 to 20°C. 4-Chloropentyl acetate (13: R² = CH₃, X¹ = Cl) (453.84 g, 2.73 mol, purity 99.03%) obtained in Example 1 was then added dropwise to the reactor at 15 to 40°C. After the completion of the dropwise addition, the reaction mixture was stirred for 6 hours at 25 to 35°C. 20% by mass hydrochloric acid (1,343.73 g with 7.37 mol of hydrogen chloride) was then added to the reaction mixture, followed by phase separation. 4-Chloro-1-pentanol (15: X¹ = Cl) (376.05 g, 2.43 mol, purity 79.16%) was obtained with a crude yield of 80.11% by concentrating the organic layer thus obtained at a reduced pressure.

The following is the spectrum data of 4-chloro-1-pentanol (15: X¹ = Cl) thus obtained.

Nuclear magnetic resonance spectrum: ¹H-NMR (500 MHz, CDCl₃): δ = 1.51 (3H, J = 6.5 Hz), 1.61-1.87 (4H, m), 2.58 (1H, br.s), 3.66 (2H, t, J = 6.1 Hz), 4.02-4.09 (1H, m); ¹³C-NMR (500 MHz, CDCl₃): δ = 25.36, 29.63, 36.54, 58.58, 62.15.

Mass spectrum: EI-mass spectrum (70 eV): m/z 104 (M⁺-18), 89, 76, 68, 56, 41. Infrared absorption spectrum: (D-ATR): v = 3331, 2971, 2947, 2873, 1446, 1380, 1258, 1193, 1127, 1060, 1029, 1011, 982, 899, 671, 611.

### Example 3: Preparation of 4-chloropentyl methoxymethyl ether (1A: R¹ = H, X¹ = Cl)

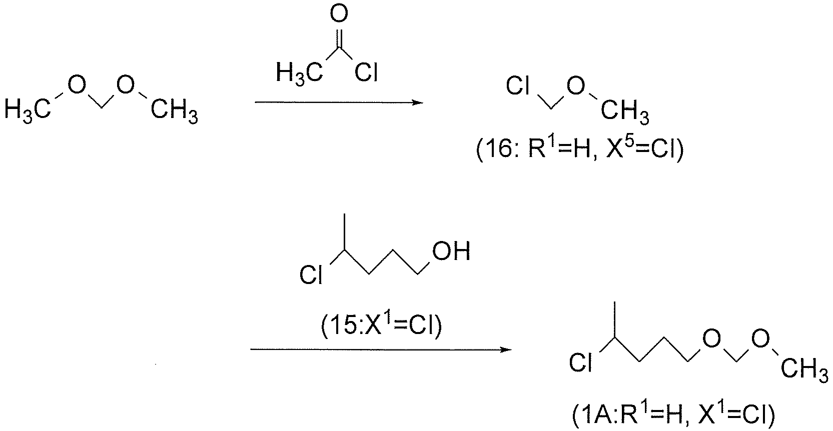

Dimethoxymethane (271.58 g, 3.57 mol) and zinc chloride (3.31 g, 0.024 mol) were placed in a reactor at a room temperature and stirred for 3 minutes at 15 to 25°C. Acetyl chloride (247.78 g, 3.16 mol) was then added dropwise to the reactor at 30 to 40°C. After the completion of the dropwise addition, chloromethyl methyl ether (16: R¹ = H, X⁵ = Cl) was formed by stirring for 2 hours at 35 to 45°C.

Then, *N*,*N*-diethylaniline (615.96 g, 4.13 mol) and 4-chloro-1-pentanol (15: X¹ = Cl) (376.05 g, 2.43 mol, purity 79.16%) obtained in Example 2 were added dropwise to the aforesaid reactor at 20 to 30°C. After the completion of the dropwise addition, the reaction mixture was stirred for 4 hours at 20 to 30°C. An aqueous solution of 25% by mass sodium hydroxide (842.47 g with 5.27 mol of sodium hydroxide) and water (535.04 g) were then added to the reaction mixture, followed by phase separation. The organic layer thus obtained was washed three times with 20% by mass hydrochloric acid (336.23 g, 1.84 mol). Continuing, washing with an aqueous solution of 25% by mass sodium hydroxide (152.60 g with 0.97 mol of sodium hydroxide) and then with methanol (40.97 g, 1.28 mol) were sequentially carried out, followed by phase separation. The organic layer thus obtained was subjected to distillation at a reduced pressure to obtain 4-chloropentyl methoxymethyl ether (1: *n =* 1, R¹ = H, X¹ = Cl) (387.39 g, 2.27 mol, purity 97.61%, b.p. = 68.1 to 77.9°C/0.67 kPa (5.0 mmHg)) with a yield of 83.11%.

The following is the spectrum data of 4-chloropentyl methoxymethyl ether (1A: R¹ = H, X¹ = Cl) thus obtained.

Nuclear magnetic resonance spectrum: ¹H-NMR (500 MHz, CDCl₃): δ = 1.52 (3H, J = 6.5 Hz), 1.61-1.88 (4H, m), 3.35 (3H, s), 3.51-3.58 (2H, m), 4.01-4.09 (1H, m), 4.60 (2H, s); ¹³C-NMR (500 MHz, CDCl₃): δ = 25.36, 26.90, 37.04, 55.13, 58.50, 67.02, 96.37.

Mass spectrum: EI-mass spectrum (70 eV): m/z 165 (M⁺-1), 151, 136, 120, 104, 85, 69, 45.

Infrared absorption spectrum: (D-ATR): v = 2930, 2883, 1446, 1381, 1261, 1215, 1152, 1110, 1080, 1044, 919, 610.

### Example 4: Preparation of 6-hydroxy-4-methylheptyl methoxymethyl ether (4: R¹ = H)

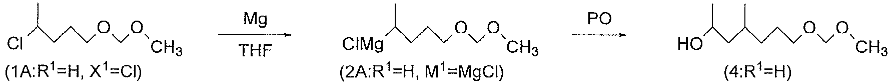

Magnesium (89.30 g, 3.68 gram atoms) and tetrahydrofuran (1,050.00 g) were placed in a reactor at a room temperature and stirred for 21 minutes at 60 to 65°C. 4-Chloropentyl methoxymethyl ether (1A: R¹ = H, X¹ = Cl) (583.28 g, 3.50 mol, purity 100%) prepared according to Example 3 was then added dropwise to the reactor at 60 to 75°C. After the completion of the dropwise addition, 4-methoxymethoxy-1-methylbutylmagnesium chloride (2A: R¹ = H, M^{1A} = MgCl) was formed by stirring for 4 hours at 75 to 80°C.

Then, the internal temperature of the aforesaid reactor was cooled to 0 to 10°C. Continuing, cuprous chloride (0.73 g, 0.0070 mol) was added to the reactor, and then propylene oxide (233.77 g, 4.02 mol) was added dropwise at 5 to 30°C. After the completion of the dropwise addition, the reaction mixture was stirred for 1.5 hours at 15 to 25°C. An aqueous solution of acetic acid (prepared from acetic acid (477.65 g) and water (1,433.19 g)) and hexane (339.68 g) were then added to the reaction mixture, followed by phase separation. The organic layer thus obtained was concentrated at a reduced pressure. The resulting concentrate was then subjected to distillation at a reduced pressure to obtain 6-hydroxy-4-methylheptyl methoxymethyl ether (4: R¹ = H) (579.26 g, 2.99 mol, purity 98.25%, b.p. = 107.0 to 111.5°C/0.049 kPa (0.37 mmHg)) with a yield of 85.45%.

The following is the spectrum data of 6-hydroxy-4-methylheptyl methoxymethyl ether (4: R¹ = H) thus obtained.

Nuclear magnetic resonance spectrum: ¹H-NMR (500 MHz, CDCl₃): δ = 0.89 (3H, dd, J = 6.7 Hz, 3.4 Hz), 1.10-1.70 (8H, m), 1.16 (3H, dd, J = 6.9 Hz, 6.1 Hz), 3.33 (3H, J = 1.2 Hz), 3.46-3.52 (2H, m), 3.83-3.91 (1H, m), 4.59 (2H, J = 1.2 Hz); ¹³C-NMR (500 MHz, CDCl₃): δ = 19.30, 20.01, 23.69, 24.32, 27.01, 27.06, 29.22, 29.55, 33.11, 34.00, 46.69, 46.86, 55.05, 65.66, 65.97, 68.05, 68.09, 96.32, 96.34.

Mass spectrum: EI-mass spectrum (70 eV): m/z 190 (M⁺), 176, 159, 127, 109, 95, 85, 69, 55, 45.

Infrared absorption spectrum: (D-ATR): v = 3422, 2928, 1460, 1377, 1305, 1215, 1153, 1110, 1046, 920, 626.

### Example 5: Preparation of 6-chloro-4-methylheptyl methoxymethyl ether (1B: R¹ = H, X¹ = Cl)

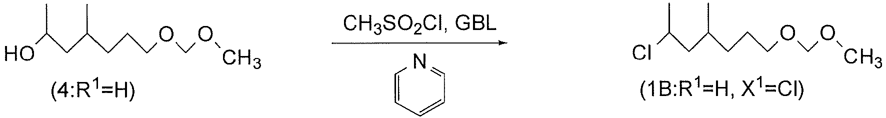

6-Hydroxy-4-methylheptyl methoxymethyl ether (4: R¹ = H) (555.14 g, 2.87 mol, purity 98.25%) obtained in Example 4, pyridine (340.05 g, 4.30 mol), and GBL (429.90 g) were placed in a reactor and stirred for 21 minutes at 35 to 45°C.

Methanesulfonyl chloride (CH₃SO₂Cl) (393.96 g, 3.44 mol) was then added dropwise at 35 to 55°C. After the completion of the dropwise addition, the reaction mixture was heated to 60 to 65°C and stirred for 9 hours. After the completion of the stirring, water (716.50 g) and hexane (429.90 g) were added, followed by phase separation. The aqueous layer was removed to obtain the organic layer. The organic layer thus obtained was washed with an aqueous solution of acetic acid (prepared from acetic acid (30.16 g) and water (377.10 g)), followed by washing with an aqueous solution of sodium bicarbonate (prepared from sodium bicarbonate (15.08 g) and water (377.10 g)). The organic layer thus obtained was concentrated at a reduced pressure. The resulting concentrate was then subjected to distillation at a reduced pressure to obtain 6-chloro-4-methylheptyl methoxymethyl ether (1B: R¹ = H, X¹ = Cl) (486.24 g, 2.07 mol, purity 88.86%, b.p. = 86.0 to 95.4°C/0.29 kPa (2.2 mmHg)) with a yield of 72.23%.

The following is the spectrum data of 6-chloro-4-methylheptyl methoxymethyl ether (1B: R¹ = H, X¹ = Cl) thus obtained.

Nuclear magnetic resonance spectrum: ¹H-NMR (500 MHz, CDCl₃): δ = 0.90 (3H, dd, J = 6.7 Hz, 3.9 Hz), 1.10-1.45 (2H, m), 1.45-1.53 (3H, m), 1.53-1.80 (5H, m), 3.35 (3H, J = 0.75 Hz), 3.50 (2H, t, J = 6.9 Hz), 4.05-4.14 (1H, m), 4.61 (2H, s); ¹³C-NMR (500 MHz, CDCl₃): δ = 18.68, 19.59, 25.37, 26.08, 26.90, 27.06, 30.10, 30.20, 32.43, 33.69, 47.60, 47.90, 55.09, 56.65, 56.87, 67.93, 67.99, 96.38, 96.41.

Mass spectrum: EI-mass spectrum (70 eV): m/z 207 (M⁺-1), 193, 177, 163, 147, 127, 111, 95, 82, 69, 45.

Infrared absorption spectrum: (D-ATR): v = 2929, 2879, 1455, 1380, 1214, 1152, 1111, 1047, 919, 675, 615.

### Example 6: Preparation of 6-chloro-4-methylheptyl butyloxymethyl ether (1B: R¹ = CH₂CH₂CH₃, X¹ = Cl)

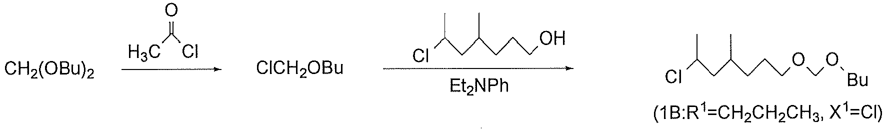

Dibutoxymethane (3.75 g, 0.023 mol) and zinc chloride (0.02 g, 0.00018 mol) were placed in a reactor at a room temperature and stirred for 18 minutes at 15 to 25°C. Acetyl chloride (1.62 g, 0.021 mol) was then added dropwise to the reactor at 30 to 40°C. After the completion of the dropwise addition, the reaction mixture was stirred for 6 hours at 38 to 42°C to obtain chloromethyl butyl ether.

A mixed solution of *N*,*N*-diethylaniline (3.09 g, 0.021 mol) and 6-chloro-4-methylheptanol (3.00 g, 0.018 mol) was then added dropwise to the reactor at 20 to 30°C. After the completion of the dropwise addition, the reaction mixture was stirred for 17 hours at 20 to 30°C. An aqueous solution of 25% by mass sodium hydroxide (3.12 g) and water (1.98 g) were then added to the reaction mixture, followed by phase separation. The organic layer thus obtained was concentrated at a reduced pressure. The resulting concentrate was then subjected to distillation at a reduced pressure to obtain 6-chloro-4-methylheptyl butyloxymethyl ether (1B: R¹ = CH₂CH₂CH₃, X¹ = Cl) (3.30 g, 0.012 mol, purity 92.84%, b.p. = 95.0 to 97.1 °C/0.085 kPa (0.64 mmHg)) with a yield of 67.99%.

The following is the spectrum data of 6-chloro-4-methylheptyl butyloxymethyl ether (1B: R¹ = CH₂CH₂CH₃, X¹ = Cl) thus obtained.

Nuclear magnetic resonance spectrum: ¹H-NMR (500 MHz, CDCl₃): δ = 0.88-0.94 (6H, m), 1.10-1.80 (14H, m), 3.51 (2H, t, J = 6.9 Hz), 3.52 (2H, t, J = 6.9 Hz), 4.05-4.14 (1H, m), 4.66 (2H, s); ¹³C-NMR (500 MHz, CDCl₃): δ = 13.86, 18.68, 19.36, 19.60, 25.36, 26.09, 26.91, 27.08, 30.11, 30.21, 31.79, 32.47, 33.72, 47.58, 47.90, 56.65, 56.88, 67.53, 67.55, 67.91, 67.98, 95.26, 95.29.

Mass spectrum: EI-mass spectrum (70 eV): m/z 249 (M⁺-1), 177, 141, 111, 87, 57.

Infrared absorption spectrum: (D-ATR): v = 2958, 2931, 2873, 1458, 1380, 1115, 1072, 1048, 826, 739, 676, 617.

### Example 7: Preparation of 6-chloro-4-methylheptyl benzyloxymethyl ether (1B: R¹ = Ph, X¹ = Cl)

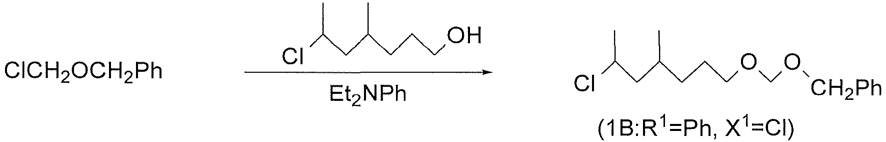

Benzyl chloromethyl ether (3.60 g, 0.021 mol, purity 90.00%), and toluene (4.00 g) were placed a reactor at a room temperature and stirred for 11 minutes at 15 to 25°C. A mixed solution of *N*,*N*-diethylaniline (3.09 g, 0.021 mol) and 6-chloro-4-methylheptanol (3.00 g, 0.018 mol) was then added dropwise to the reactor at 20 to 30°C. After the completion of the dropwise addition, the reaction mixture was stirred for 24 hours at 20 to 30°C. An aqueous solution of 25% by mass sodium hydroxide (4.07 g) and water (2.58 g) were then added to the reaction mixture, followed by phase separation. The organic layer thus obtained was concentrated at a reduced pressure. The resulting concentrate was then subjected to distillation at a reduced pressure to obtain 6-chloro-4-methylheptyl benzyloxymethyl ether (1B: R¹ = Ph, X¹ = Cl) (4.38 g, 0.014 mol, purity 89.72%, b.p. = 131.1 to 134.8°C/0.043 kPa (0.32 mmHg)) with a yield of 76.68%.

The following is the spectrum data of 6-chloro-4-methylheptyl benzyloxymethyl ether (1B: R¹ = Ph, X¹ = Cl) thus obtained.

Nuclear magnetic resonance spectrum: ¹H-NMR (500 MHz, CDCl₃): δ = 0.92 (3H, q, J = 3.4 Hz), 1.54-1.82 (10H, m), 3.59 (2H, t, J = 6.9 Hz), 4.07-4.16 (1H, m), 4.61 (2H, J = 1.2 Hz), 4.76 (2H, s), 7.27-7.33 (1H, m), 7.33-7.38 (4H, m); ¹³C-NMR (500 MHz, CDCl₃): δ = 18.69, 19.61, 25.37, 26.09, 26.89, 27.05, 30.12, 30.21, 32.42, 33.70, 47.56, 47.87, 56.66, 56.89, 68.20, 68.23, 69.26, 69.28, 94.62, 94.65, 127.63, 127.81, 127.84, 128.38, 137.94.

Mass spectrum: EI-mass spectrum (70 eV): m/z 284 (M⁺), 193, 147, 120, 91, 69. Infrared absorption spectrum: (D-ATR): v = 3031, 2929, 2872, 1454, 1379, 1207, 1168, 1113, 1049, 1028, 736, 698, 614.

### Example 8: Preparation of 4,6-dimethyloctadecyl methoxymethyl ether (6: R¹ = H)

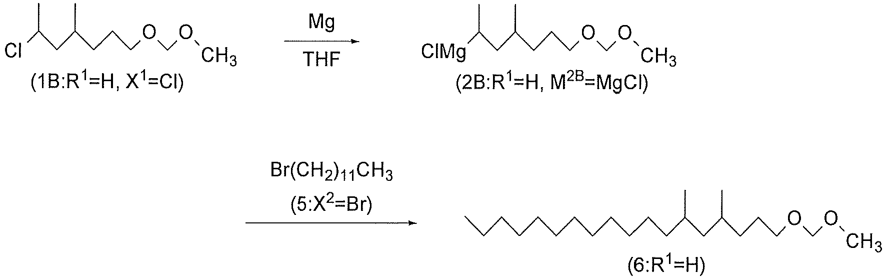

Magnesium (8.69 g, 0.36 gram atoms) and tetrahydrofuran (102.18 g) were placed in a reactor at a room temperature and stirred for 12 minutes at 60 to 65°C. 6-Chloro-4-methylheptyl methoxymethyl ether (1B: R¹ = H, X¹ = Cl) (80.01 g, 0.34 mol, purity 88.86%) obtained in Example 5 was then added dropwise to the reactor at 60 to 75°C. After the completion of the dropwise addition, the reaction mixture was stirred for 3 hours at 75 to 80°C to obtain 6-methoxymethoxy-1,3-dimethylhexylmagnesium chloride (2B: R¹ = H, M^{2B} = MgCl).

Then, cuprous chloride (0.38 g, 0.0038 mol), triethyl phosphite (3.82 g, 0.023 mol), tetrahydrofuran (100.00 g), and 1-bromododecane (5: X² = Br) (84.89 g, 0.34 mol) were placed in another reactor, to which the aforesaid 6-methoxymethoxy-1,3-dimethylhexylmagnesium chloride (2B: R¹ = H, M^{2B} = MgCl) prepared above was added dropwise at 5 to 20°C. After the completion of the dropwise addition, the reaction mixture was stirred for 2 hours at 15 to 25°C. An aqueous solution of acetic acid (prepared from acetic acid (5.41 g) and water (93.84 g)) and an aqueous solution of 25% by mass sodium hydroxide (7.13 g) were then added to the reaction mixture, followed by phase separation. The organic layer thus obtained was concentrated at a reduced pressure. The resulting concentrate was then subjected to distillation at a reduced pressure to obtain 4,6-dimethyloctadecyl methoxymethyl ether (6: R¹ = H) (92.93 g, 0.24 mol, purity 90.10%, b.p. = 149.0 to 161.1°C/0.061 kPa (0.46 mmHg)) with a yield of 71.74%.

The following is the spectrum data of 4,6-dimethyloctadecyl methoxymethyl ether (6: R¹ = H) thus obtained.

Nuclear magnetic resonance spectrum: ¹H-NMR (500 MHz, CDCl₃): δ = 0.79-0.86 (6H, m), 0.88 (3H, t, J = 6.9 Hz), 1.00-1.70 (30H, m), 3.36 (3H, s), 3.50 (2H, t, J = 6.9 Hz), 4.62 (2H, s); ¹³C-NMR(500 MHz, CDCl₃): δ = 14.10, 19.40, 19.48, 20.17, 20.28, 22.69, 26.91, 27.06, 27.15, 27.30, 29.36, 29.65, 29.70, 29.73, 29.90, 29.97, 30.00, 30.01, 30.05, 31.92, 33.22, 34.31, 36.83, 37.99, 44.78, 45.11, 55.06, 68.24, 68.30, 96.39.

Mass spectrum: EI-mass spectrum (70 eV): m/z 341 (M⁺-1), 297, 237, 196, 167, 139, 111, 85, 69, 45.

Infrared absorption spectrum: (D-ATR): v = 2924, 2853, 1465, 1378, 1153, 1112, 1047, 921, 722.

### Example 9: Preparation of 4,6-dimethyloctadecanol (7)

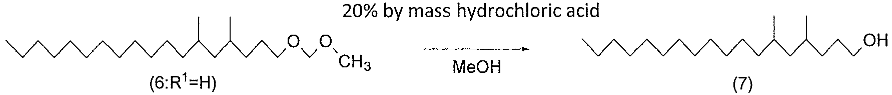

4,6-Dimethyloctadecyl methoxymethyl ether (6: R¹ = H) (75.13 g, 0.20 mol, purity 90.10%) obtained in Example 8, methanol (98.80 g, 3.08 mol), and 20% by mass hydrochloric acid (9.88 g with 0.054 mol of hydrogen chloride) were placed in a reactor equipped with a distillation tower. The reaction mixture was heated to 60°C and stirred for 19 minutes. After the completion of the stirring, the internal temperature of the reactor was heated to 65 to 70°C to distill off a mixture of by-produced dimethoxymethane and methanol from the distillation tower. The reaction mixture was sampled during the reaction. After the conversion was confirmed to be 100%, water (59.28 g) was added to the reaction mixture, followed by phase separation. The aqueous layer was removed to obtain the organic layer. The organic layer thus obtained was concentrated at a reduced pressure. The resulting concentrate was then subjected to distillation at a reduced pressure to obtain 4,6-dimethyloctadecanol (7) (63.44 g, 0.19 mol, purity 91.15%, b.p. = 141.2 to 163.1°C/0.037 kPa (0.28 mmHg)) with a yield of 98.00%.

The following is the spectrum data of 4,6-dimethyloctadecanol (7) thus obtained.

Nuclear magnetic resonance spectrum: ¹H-NMR (500 MHz, CDCl₃): δ = 0.79-0.86 (6H, m), 0.88 (3H, t, J = 6.9 Hz), 1.00-1.66 (31H, m), 3.62 (2H, t, J = 6.9 Hz); ¹³C-NMR (500 MHz, CDCl₃): δ = 14.09, 19.44, 19.47, 20.20, 20.24, 22.68, 26.92, 27.05, 29.35, 29.65, 29.69, 29.72, 29.89, 29.97, 30.00, 30.04, 30.19, 30.33, 31.91, 32.72, 33.83, 36.85, 37.97, 44.75, 45.08, 63.43, 63.47.

Mass spectrum: EI-mass spectrum (70 eV): m/z 297 (M⁺-1)280, 266, 252, 237, 210, 196, 182, 168, 154, 125, 111, 97, 83, 69, 55, 41.

Infrared absorption spectrum: (D-ATR): vmax = 3321, 2955, 2924, 2853, 1465, 1378, 1057, 721.

### Example 10: Preparation of 1-chloro-4,6-dimethyloctadecane (8: X³ = Cl)

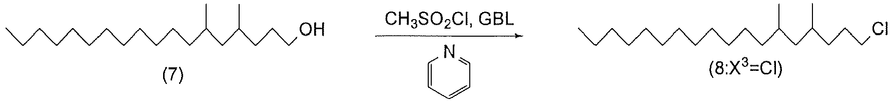

4,6-Dimethyloctadecanol (7) (63.23 g, 0.19 mol, purity 91.15%) obtained in Example 9, pyridine (22.90 g, 0.29 mol), and GBL (63.23 g) were placed in a reactor and stirred for 22 minutes at 40°C.

Methanesulfonyl chloride (CH₃SO₂Cl) (26.53 g, 0.23 mol) was then added dropwise at 40 to 60°C. After the completion of the dropwise addition, the reaction mixture was heated to 60 to 65°C and stirred for 9 hours. After the completion of the stirring, water (60.00 g) and hexane (60.00 g) were added to the reaction mixture, followed by phase separation. The aqueous layer was removed to obtain the organic layer. The organic layer thus obtained was washed with an aqueous solution of acetic acid (prepared from acetic acid (2.03 g) and water (60.00 g)), followed by washing with an aqueous solution of sodium bicarbonate (prepared from sodium bicarbonate (1.02 g) and water (60.00 g)). The organic layer thus obtained was concentrated at a reduced pressure. The resulting concentrate was then subjected to distillation at a reduced pressure to obtain 1-chloro-4,6-dimethyloctadecane (8: X³ = Cl) (61.12 g, 0.18 mol, purity 91.65%, b.p. = 166.1 to 172.5°C/0.40 kPa (3.0 mmHg)) with a yield of 91.55%.

The following is the spectrum data of 1-chloro-4,6-dimethyloctadecane (8: X³ = Cl) thus obtained.

Nuclear magnetic resonance spectrum: ¹H-NMR (500 MHz, CDCl₃): δ = 0.80-0.91 (9H, m), 1.00-1.60 (28H, m), 1.68-1.86 (2H, m), 3.52 (2H, t, J = 6.9 Hz); ¹³C-NMR (500 MHz, CDCl₃): δ = 14.11, 19.39, 19.49, 20.15, 20.22, 22.70, 26.91, 27.04, 29.37, 29.58, 29.66, 29.70, 29.73, 29.99, 30.01, 30.04, 30.18, 30.33, 31.93, 33.96, 35.06, 36.84, 37.90, 44.67, 44.97, 45.51, 45.53.

Mass spectrum: EI-mass spectrum (70 eV): m/z 301 (M⁺-15), 239, 197, 147, 111, 85, 57.

Infrared absorption spectrum: (D-ATR): vmax = 2956, 2924, 2853, 1464, 1378, 723, 656.

### Example 11: Preparation of 13,15-dimethylheptacosane (11)

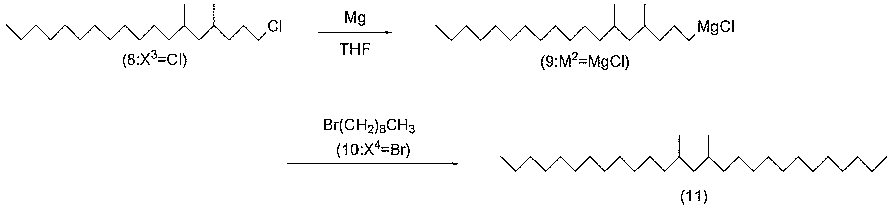

Magnesium (2.21 g, 0.091 gram atoms) and tetrahydrofuran (90.00 g) were placed in a reactor at a room temperature and stirred for 28 minutes at 60 to 65°C. 1-Chloro-4,6-dimethyloctadecane (8: X³ = Cl) (30.00 g, 0.087 mol, purity 91.65%) obtained in Example 10 was then added dropwise to the reactor at 60 to 75°C. After the completion of the dropwise addition, the reaction mixture was stirred for 4 hours at 75 to 80°C to obtain 4,6-dimethyloctadecylmagnesium chloride (9: M² = MgCl).

Then, cuprous chloride (0.10 g, 0.00098 mol), triethyl phosphite (0.97 g, 0.0059 mol), lithium chloride (0.07 g, 0.0016 mol), tetrahydrofuran (100.00 g), and 1-bromononane (10: X⁴ = Br) (17.97 g, 0.087 mol) were placed in another reactor, to which the aforesaid 4,6-dimethyloctadecylmagnesium chloride (9: M² = MgCl) prepared above was added dropwise at 15 to 25°C. After the completion of the dropwise addition, the reaction mixture was stirred for 2 hours at 20 to 30°C. An aqueous solution of acetic acid (prepared from acetic acid (0.87 g) and water (23.90 g)), 20% by mass hydrochloric acid (1.82 g), and an aqueous solution of 25% by mass sodium hydroxide (1.82 g) were then added to the reaction mixture, followed by phase separation. The organic layer thus obtained was concentrated at a reduced pressure. The resulting concentrate was then subjected to distillation at a reduced pressure to distill off low-boiling impurities. The resulting concentrate was then purified by silica gel column chromatography (hexane = 100) to obtain 13,15-dimethylheptacosane (11) (27.13 g, 0.058 mol, purity 87.87%) with a yield of 67.24%.

The following is the spectrum data of 13,15-dimethylheptacosane (11) thus obtained.

Nuclear magnetic resonance spectrum: ¹H-NMR (500 MHz, CDCl₃): δ = 0.88 (6H, t, J = 6.9 Hz), 0.82 (6H, dd, J = 10.9 Hz, 6.9 Hz), 1.01-1.07 (2H, m), 1.16-1.34 (46H, m); ¹³C-NMR (500 MHz, CDCl₃): δ = 14.12, 19.57, 20.32, 22.71, 26.92, 27.09, 29.38, 29.68, 29.73, 29.76, 30.01, 30.03, 30.07, 31.95, 36.90, 37.99.

Mass spectrum: EI-mass spectrum (70 eV): m/z 393 (M⁺-15), 365, 295, 267, 239, 196, 169, 141, 113, 85, 57, 29.

Infrared absorption spectrum: (D-ATR): v = 2956, 2923, 2853, 1466, 1377, 721.

### Example 12: Preparation of 6-bromo-4-methylheptyl methoxymethyl ether (1B: R¹ = H, X¹ = Br)

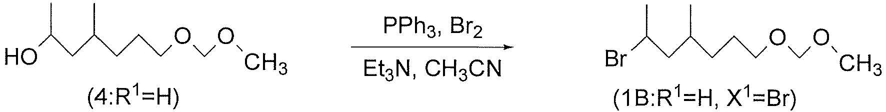

Triphenylphosphine (PPh₃) (5.51 g, 0.021 mol) and acetonitrile (CH₃CN) (15.45 g) were placed in a reactor at a room temperature and stirred for 26 minutes at 0 to 10°C. Bromine (Br₂) (3.21 g, 0.020 mol) was then added dropwise to the reactor at 0 to 10°C. After the completion of the dropwise addition, the reaction mixture was stirred for 3 hours. A mixed solution of 6-hydroxy-4-methylheptyl methoxymethyl ether (4: R¹ = H) (2.93 g, 0.015 mol, purity 98.25%) obtained in Example 4 and triethylamine (2.14 g, 0.021 mol) was then added dropwise at 0 to 10°C. After the completion of the dropwise addition, the reaction mixture was stirred for 13 hours at 15 to 25°C. An aqueous solution of acetic acid (4.33 g) and hexane (6.60 g) were then added to the reaction mixture, followed by phase separation. The organic layer thus obtained was concentrated at a reduced pressure. The resulting concentrate was then purified by silica gel column chromatography (hexane : ethyl acetate = 70 : 1 to 5 : 1) to obtain 6-bromo-4-methylheptyl methoxymethyl ether (1B: R¹ = H, X¹ = Br) (0.38 g, 0.00080 mol, purity 53.50%) with a yield of 5.31%.

The following is the spectrum data of 6-bromo-4-methylheptyl methoxymethyl ether (1B: R¹ = H, X¹ = Br) thus obtained.

Nuclear magnetic resonance spectrum: ¹H-NMR (500 MHz, CDCl₃): δ = 0.87-0.94 (3H, m), 1.10-1.96 (10H, m), 3.36 (3H, s), 3.47-3.54 (2H, m), 4.14-4.25 (1H, m), 4.61 (2H, s); ¹³C-NMR (500 MHz, CDCl₃): δ = 18.61, 18.63, 19.43, 19.45, 26.40, 26.88, 27.06, 27.18, 30.03, 30.83, 31.20, 31.31, 32.32, 33.57, 33.97, 48.36, 48.53, 48.73, 49.29, 49.53, 50.18, 67.93, 96.39, 96.43.

Mass spectrum: EI-mass spectrum (70 eV): m/z 253 (M⁺+1), 221, 191, 164, 141, 111, 69, 45.

Infrared absorption spectrum: (D-ATR): v = 2955, 2925, 2872, 1454, 1380, 1243, 1210, 11444, 1111, 1046, 919, 622, 540.

## Claims

1. A process for preparing a haloalkyl alkoxymethyl ether compound of the following general formula (1B): 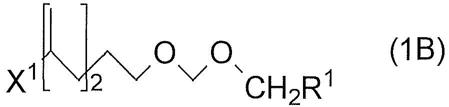
wherein X¹ represents a halogen atom, and R¹ represents a hydrogen atom, an n-alkyl group having 1 to 9 carbon atoms, or a phenyl group,
the process comprising the steps of
converting a haloalkyl alkoxymethyl ether compound of the following general formula (1A): 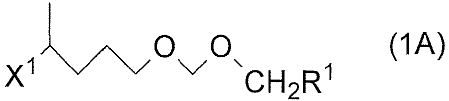
wherein X¹ and R¹ are as defined above,
into a nucleophilic reagent, 4-alkoxymethoxy-1-methylbutyl, of the following general formula (2A): 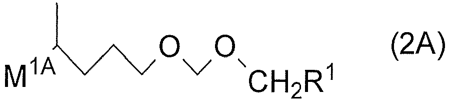
wherein M^{1A} represents Li, MgZ^{1A}, CuZ^{1A}, or CuLiZ^{1A}, Z^{1A} represents a halogen atom or a 4-alkoxymethoxy-1-methylbutyl group, and R¹ is as defined above,
subsequently subjecting the nucleophilic reagent, 4-alkoxymethoxy-1-methylbutyl (2A), to a nucleophilic addition reaction with propylene oxide of the following formula (3): 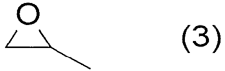
to obtain 6-hydroxy-4-methylheptyl alkoxymethyl ether compound of the following general formula (4): 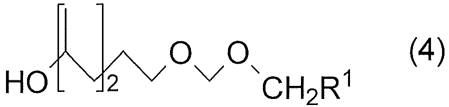
wherein R¹ is as defined above,
and
subjecting the 6-hydroxy-4-methylheptyl alkoxymethyl ether compound (4) to a halogenation reaction to form the aforesaid haloalkyl alkoxymethyl ether compound (1B).

2. A process for preparing 4,6-dimethyloctadecanol of the following formula (7): 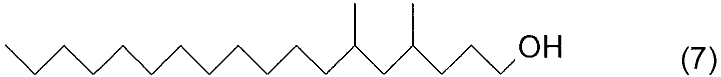 the process comprising the steps of
the process for preparing the aforesaid haloalkyl alkoxymethyl ether compound (1B) according to claim 1,
converting the haloalkyl alkoxymethyl ether compound (1B) into a nucleophilic reagent, 6-alkoxymethoxy-1,3-dimethylhexyl, of the following general formula (2B): 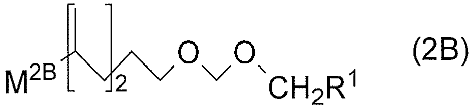
wherein M^{2B} represents Li, MgZ^{2B}, CuZ^{2B}, or CuLiZ^{2B}, Z^{2B} represents a halogen atom or a 6-alkoxymethoxy-1,3-dimethylhexyl group, and R¹ is as defined above,
and subsequently subjecting the nucleophilic reagent, 6-alkoxymethoxy-1,3-dimethylhexyl (2B), to a coupling reaction with a 1-halododecane compound of the following general formula (5):
X²(CH₂)₁₁CH₃ (5)
wherein X² represents a halogen atom,
to obtain a 4,6-dimethyloctadecyl alkoxymethyl ether compound of the following general formula (6): 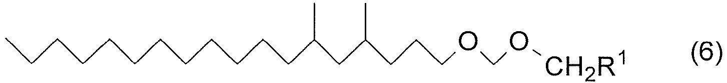
wherein R¹ is as defined above,
and
subjecting the 4,6-dimethyloctadecyl alkoxymethyl ether compound (6) to a dealkoxymethylation reaction to form the aforesaid 4,6-dimethyloctadecanol (7).

3. A process for preparing 13,15-dimethylheptacosane of the following general formula (11): 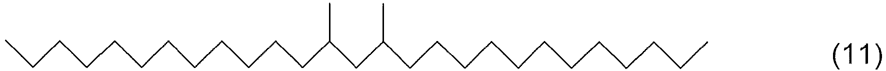 the process comprising the steps of
the process for preparing the aforesaid haloalkyl alkoxymethyl ether compound (1B) according to claim 1,
converting the haloalkyl alkoxymethyl ether compound (1B) into a nucleophilic reagent, 6-alkoxymethoxy-1,3-dimethylhexyl, of the following general formula (2B): 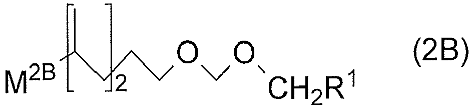 wherein M^{2B} represents Li, MgZ^{2B}, CuZ^{2B}, or CuLiZ^{2B}, Z^{2B} represents a halogen atom or a 6-alkoxymethoxy-1,3-dimethylhexyl group, and R¹ is as defined above,
and subsequently subjecting the nucleophilic reagent, 6-alkoxymethoxy-1,3-dimethylhexyl (2B), to a coupling reaction with a 1-halododecane compound of the following general formula (5):
X² (CH₂)₁₁CH₃ (5)
wherein X² represents a halogen atom,
to obtain a 4,6-dimethyloctadecyl alkoxymethyl ether compound of the following general formula (6): 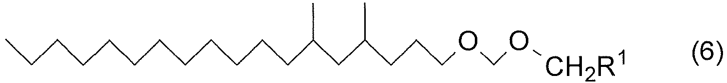
wherein R¹ is as defined above,
subjecting the 4,6-dimethyloctadecyl alkoxymethyl ether compound (6) to a dealkoxymethylation reaction to obtain 4,6-dimethyloctadecanol of the following formula (7): 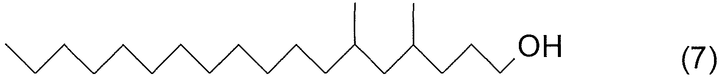
subjecting the 4,6-dimethyloctadecanol (7) to a halogenation reaction to obtain a 1-halo-4,6-dimethyloctadecane compound of the following general formula (8): 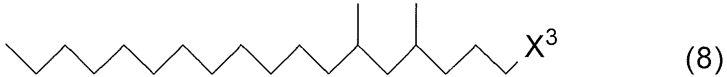
wherein X³ represents a halogen atom,
converting the 1-halo-4,6-dimethyloctadecane compound (8) into a nucleophilic reagent, 4,6-dimethyloctadecyl, of the following general formula (9): 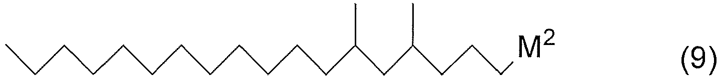
wherein M² represents Li, MgZ², CuZ², or CuLiZ², and Z² represents a halogen atom or a 4,6-dimethyloctadecyl group,
and subsequently subjecting the nucleophilic reagent, 4,6-dimethyloctadecyl (9), to a coupling reaction with a 1-halononane compound of the following general formula (10):
X⁴(CH₂)₈CH₃ (10)
wherein X⁴ represents a halogen atom,
to form the aforesaid 13,15-dimethylheptacosane (11).

4. The process for preparing a haloalkyl alkoxymethyl ether compound (1B) according to claim 1, the process further comprising the steps of
opening the tetrahydrofuran ring of 2-methyltetrahydrofuran of the following formula: 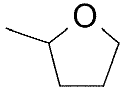
with an acid halide of the following general formula (12): 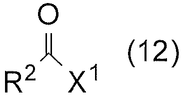
wherein R² represents an alkyl group having 1 to 9 carbon atoms or a phenyl group, and X¹ is as defined above,
to obtain 4-halopentyl acylate compound of the following general formula (13): 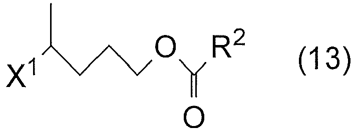
wherein X¹ and R² are as defined above,
subjecting the 4-halopentyl acylate compound (13) with the organometallic reagent of the general formula (14):
R³M³ (14)
wherein R³ represents an alkyl group having 1 to 14 carbon atoms or an ethynyl group, M³ represents Li, Na, K, Ag, MgZ⁴, or CaZ⁴, and Z⁴ represents a halogen atom or R³,
to obtain 4-halo-1-pentanol compound of the general formula (15): 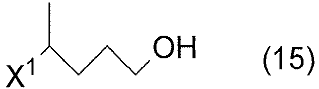
wherein X¹ is as defined above,
subjecting the 4-halo-1-pentanol compound (15) to an alkoxymethylation with a halomethyl alkyl ether compound of the following general formula (16): 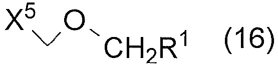
wherein R¹ is as defined above, and X⁵ represents a halogen atom,
to form a haloalkyl alkoxymethyl ether compound of the following general formula (1A): 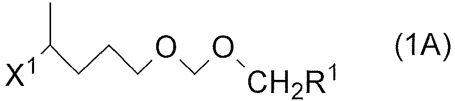
wherein X¹ and R¹ are as defined above.

5. The process for preparing 4,6-dimethyloctadecanol (7) according to claim 2, the process further comprising the steps of
opening the tetrahydrofuran ring of 2-methyltetrahydrofuran of the following formula: 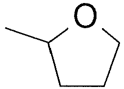
with an acid halide of the following general formula (12): 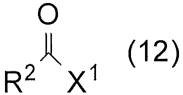
wherein R² represents an alkyl group having 1 to 9 carbon atoms or a phenyl group, and X¹ is as defined above,
to obtain 4-halopentyl acylate compound of the following general formula (13): 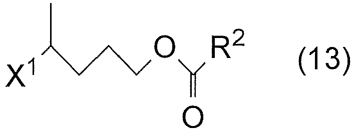
wherein X¹ and R² are as defined above,
subjecting the 4-halopentyl acylate compound (13) with the organometallic reagent of the general formula (14):
R³M³ (14)
wherein R³ represents an alkyl group having 1 to 14 carbon atoms or an ethynyl group, M³ represents Li, Na, K, Ag, MgZ⁴, or CaZ⁴, and Z⁴ represents a halogen atom or R³,
to obtain 4-halo-1-pentanol compound of the general formula (15): 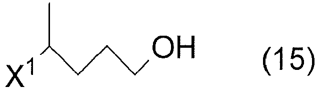
wherein X¹ is as defined above,
subjecting the 4-halo-1-pentanol compound (15) to an alkoxymethylation with a halomethyl alkyl ether compound of the following general formula (16): 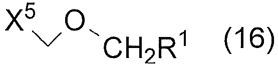
wherein R¹ is as defined above, and X⁵ represents a halogen atom,
to form a haloalkyl alkoxymethyl ether compound of the following general formula (1A): 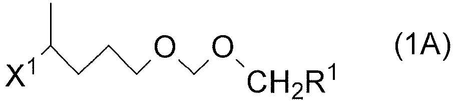
wherein X¹ and R¹ are as defined above.

6. The process for preparing 13,15-dimethylheptacosane (11) according to claim 3, the process further comprising the steps of
opening the tetrahydrofuran ring of 2-methyltetrahydrofuran of the following formula: 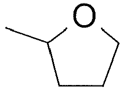
with an acid halide of the following general formula (12): 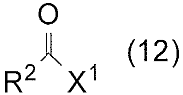
wherein R² represents an alkyl group having 1 to 9 carbon atoms or a phenyl group, and X¹ is as defined above,
to obtain 4-halopentyl acylate compound of the following general formula (13): 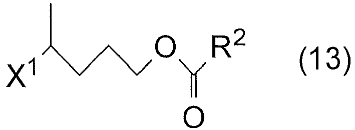
wherein X¹ and R² are as defined above,
subjecting the 4-halopentyl acylate compound (13) with the organometallic reagent of the general formula (14):
R³M³ (14)
wherein R³ represents an alkyl group having 1 to 14 carbon atoms or an ethynyl group, M³ represents Li, Na, K, Ag, MgZ⁴, or CaZ⁴, and Z⁴ represents a halogen atom or R³,
to obtain 4-halo-1-pentanol compound of the general formula (15): 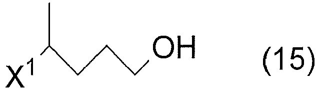
wherein X¹ is as defined above,
subjecting the 4-halo-1-pentanol compound (15) to an alkoxymethylation with a halomethyl alkyl ether compound of the following general formula (16): 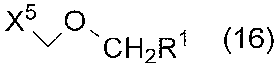
wherein R¹ is as defined above, and X⁵ represents a halogen atom,
to form a haloalkyl alkoxymethyl ether compound of the following general formula (1A): 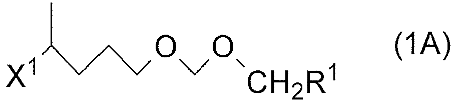
wherein X¹ and R¹ are as defined above.

7. A process for preparing 13,15-dimethylheptacosane of the following general formula (11): 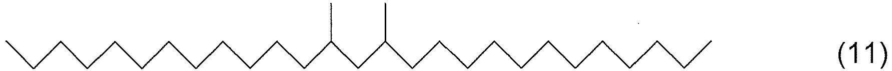 the process comprising the steps of
converting a haloalkyl alkoxymethyl ether compound of the following general formula (1B): 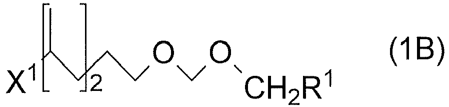
wherein X¹ represents a halogen atom, and R¹ represents a hydrogen atom, an n-alkyl group having 1 to 9 carbon atoms, or a phenyl group,
into a nucleophilic reagent, 6-alkoxymethoxy-1,3-dimethylhexyl, of the following general formula (2B): 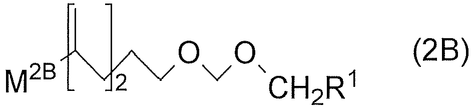
wherein M^{2B} represents Li, MgZ^{2B}, CuZ^{2B}, or CuLiZ^{2B}, Z^{2B} represents a halogen atom or a 6-alkoxymethoxy-1,3-dimethylhexyl group, and R¹ is as defined above,
subsequently subjecting the nucleophilic reagent, 6-alkoxymethoxy-1,3-dimethylhexyl (2B), to a coupling reaction with a 1-halododecane compound of the following general formula (5):
X²(CH₂)₁₁CH₃ (5)
wherein X² represents a halogen atom,
to obtain a 4,6-dimethyloctadecyl alkoxymethyl ether compound of the following general formula (6): 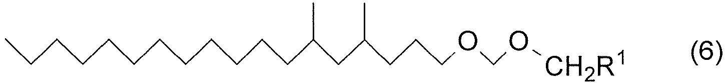
wherein R¹ is as defined above,
subjecting the 4,6-dimethyloctadecyl alkoxymethyl ether compound (6) to a dealkoxymethylation reaction to obtain 4,6-dimethyloctadecanol of the following formula (7): 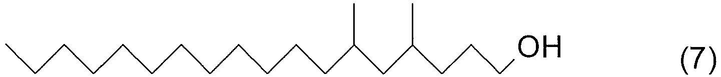
subjecting the 4,6-dimethyloctadecanol (7) to a halogenation reaction to obtain a 1-halo-4,6-dimethyloctadecane compound of the following general formula (8): 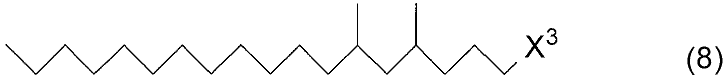
wherein X³ represents a halogen atom,
converting the 1-halo-4,6-dimethyloctadecane compound (8) into a nucleophilic reagent, 4,6-dimethyloctadecyl, of the following general formula (9): 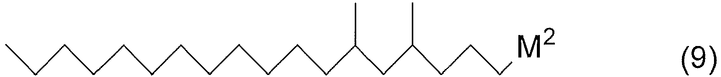 wherein M² represents Li, MgZ², CuZ², or CuLiZ², and Z² represents a halogen atom or a 4,6-dimethyloctadecyl group,
and subsequently subjecting the nucleophilic reagent, 4,6-dimethyloctadecyl (9), to a coupling reaction with a 1-halononane compound of the following general formula (10):
X⁴(CH₂)₈CH₃ (10)
wherein X⁴ represents a halogen atom,
to form the aforesaid 13,15-dimethylheptacosane (11).

8. A haloalkyl alkoxymethyl ether compound of the following general formula (1): 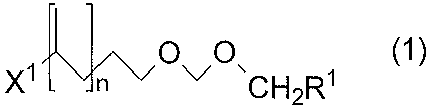 wherein X¹ represents a halogen atom, R¹ represents a hydrogen atom, an n-alkyl group having 1 to 9 carbon atoms, or a phenyl group, and n represents 1 or 2.

9. The haloalkyl alkoxymethyl ether compound (1) according to claim 8, wherein *n* is 1, which is represented by the following general formula (1A): 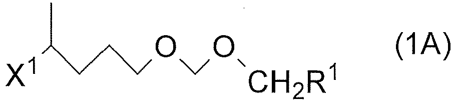 wherein X¹ represents a halogen atom, and R¹ represents a hydrogen atom, an n-alkyl group having 1 to 9 carbon atoms, or a phenyl group.

10. The haloalkyl alkoxymethyl ether compound (1) according to claim 8, wherein *n* is 2, which is represented by the following general formula (1B): 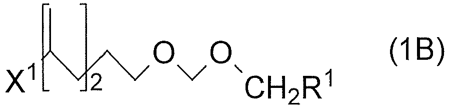 wherein X¹ represents a halogen atom, and R¹ represents a hydrogen atom, an n-alkyl group having 1 to 9 carbon atoms, or a phenyl group.

## Patentansprüche

1. Ein Verfahren zur Herstellung einer Halogenalkylalkoxymethylether-Verbindung der folgenden allgemeinen Formel (1B): 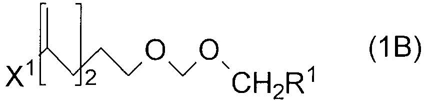
wobei X¹ ein Halogenatom dargestellt und R¹ ein Wasserstoffatom, eine n-Alkylgruppe mit 1 bis 9 Kohlenstoffatomen oder eine Phenylgruppe dargestellt,
wobei das Verfahren die Schritte umfasst:
Umwandeln einer Halogenalkylalkoxymethylether-Verbindung der folgenden allgemeinen Formel (1A): 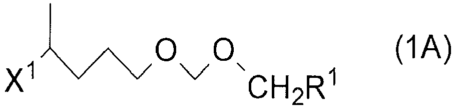
wobei X¹ und R¹ wie oben definiert sind,
in ein nukleophiles Reagenz, 4-Alkoxymethoxy-1-methylbutyl, der folgenden allgemeinen Formel (2A): 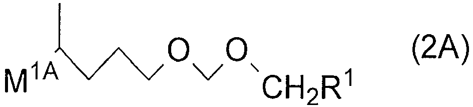
wobei M^{1A} Li, MgZ^{1A}, CuZ^{1A} oder CuLiZ^{1A} darstellt, Z^{1A} ein Halogenatom oder eine 4-Alkoxymethoxy-1-methylbutyl Gruppe darstellt, und R¹ wie oben definiert ist, anschließendes Unterziehen des nukleophilen Reagenz, 4-Alkoxymethoxy-1-methylbutyl (2A), einer nukleophilen Additionsreaktion mit Propylenoxid der folgenden Formel (3): 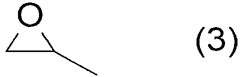
um eine 6-Hydroxy-4-methylheptylalkoxymethylether-Verbindung der folgenden allgemeinen Formel (4) zu erhalten: 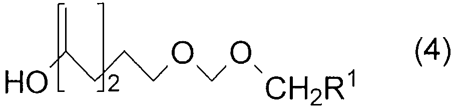
wobei R¹ wie oben definiert ist,
und
Unterziehen der 6-Hydroxy-4-methylheptylalkoxymethylether-Verbindung (4) einer Halogenierungsreaktion, um die vorgenannte Halogenalkylalkoxymethylether-Verbindung (1B) zu bilden.

2. Ein Verfahren zur Herstellung von 4,6-Dimethyloctadecanol der folgenden Formel (7): 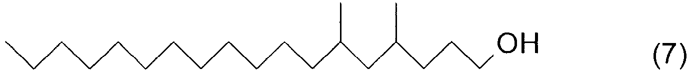 wobei das Verfahren die Schritte umfasst:
das Verfahren zur Herstellung der vorgenannten Halogenalkylalkoxymethylether-Verbindung (1B) gemäß Anspruch 1,
Umwandeln der Halogenalkylalkoxymethylether-Verbindung (1B) in ein nukleophiles Reagenz, 6-Alkoxymethoxy-1,3-dimethylhexyl, der folgenden allgemeinen Formel (2B): 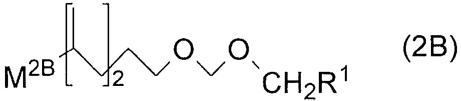 wobei M^{2B} Li, MgZ^{2B}, CuZ^{2B} oder CuLiZ^{2B} dargestellt, Z^{2B} ein Halogenatom oder eine 6-Alkoxymethoxy-1,3-dimethylhexyl Gruppe dargestellt, und R¹ wie oben definiert ist,
und anschließendes Unterziehen des nukleophilen Reagenz, 6-Alkoxymethoxy-1,3-dimethylhexyl (2B), einer Kupplungsreaktion mit einer 1-Halogendodecanverbindung der folgenden allgemeinen Formel (5):
X²(CH₂)₁₁CH₃ (5)
wobei X² ein Halogenatom dargestellt,
um eine 4,6-Dimethyloctadecylalkoxymethylether-Verbindung der folgenden allgemeinen Formel (6) zu erhalten: 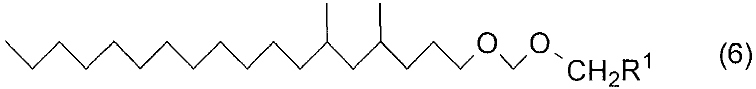
wobei R¹ wie oben definiert ist, und
Unterziehen der 4,6-Dimethyloctadecylalkoxymethylether-Verbindung (6) einer Dealkoxymethylierungsreaktion, um das vorgenannte 4,6-Dimethyloctadecanol (7) zu bilden.

3. Ein Verfahren zur Herstellung von 13,15-Dimethylheptacosan der folgenden allgemeinen Formel (11): 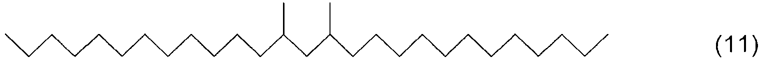 wobei das Verfahren die Schritte umfasst:
das Verfahrens zur Herstellung der vorgenannten Halogenalkylalkoxymethylether-Verbindung (1B) gemäß Anspruch 1,
Umwandeln der Halogenalkylalkoxymethylether-Verbindung (1B) in ein nukleophiles Reagenz, 6-Alkoxymethoxy-1,3-dimethylhexyl, der folgenden allgemeinen Formel (2B): 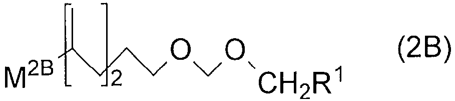
wobei M^{2B} Li, MgZ^{2B}, CuZ^{2B} oder CuLiZ^{2B} darstellt, Z^{2B} ein Halogenatom oder eine 6-Alkoxymethoxy-1,3-dimethylhexyl Gruppe darstellt, und R¹ wie oben definiert ist,
und anschließendes Unterziehen des nukleophilen Reagenz, 6-Alkoxymethoxy-1,3-dimethylhexyl (2B); einer Kupplungsreaktion mit einer 1-Halogendodecanverbindung der folgenden allgemeinen Formel (5):
X²(CH₂)₁₁CH₃ (5)
wobei X² ein Halogenatom dargestellt,
um eine 4,6-Dimethyloctadecylalkoxymethylether-Verbindung der folgenden allgemeinen Formel (6) zu erhalten: 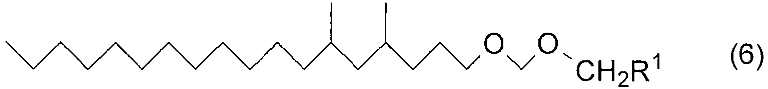
wobei R¹ wie oben definiert ist,
Unterziehen der 4,6-Dimethyloctadecylalkoxymethylether-Verbindung (6) einer Dealkoxymethylierungsreaktion, um 4,6-Dimethyloctadecanol der folgenden Formel (7) zu erhalten: 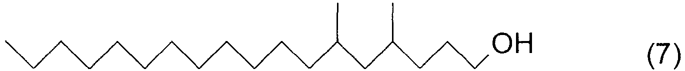
Unterziehen des 4,6-Dimethyloctadecanols (7) einer Halogenierungsreaktion, um eine 1-Halogen-4,6-dimethyloctadecan-Verbindung der folgenden allgemeinen Formel (8) zu erhalten: 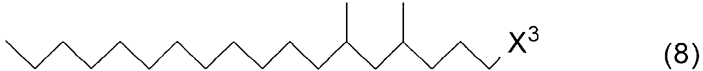 wobei X³ ein Halogenatom dargestellt,
Umwandeln der 1-Halo-4,6-dimethyloctadecan-Verbindung (8) in ein nukleophiles Reagenz, 4,6-Dimethyloctadecyl, der folgenden allgemeinen Formel (9): 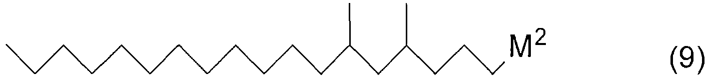 wobei M² Li, MgZ², CuZ² oder CuLiZ² darstellt, und Z² ein Halogenatom oder eine 4,6-Dimethyloctadecyl Gruppe darstellt,
und anschließendes Unterziehen des nukleophilen Reagenz, 4,6-Dimethyloctadecyl (9), einer Kupplungsreaktion mit einer 1-Halogennonanverbindung der folgenden allgemeinen Formel (10):
X⁴(CH₂)₈CH₃ (10)
wobei X⁴ ein Halogenatom darstellt,
um das vorgenannte 13,15-Dimethylheptacosan (11) zu bilden.

4. Das Verfahren zur Herstellung einer Haloalkylalkoxymethylether-Verbindung (1B) gemäß Anspruch 1, wobei das Verfahren ferner die Schritte umfasst:
Öffnen des Tetrahydrofuranrings von 2-Methyltetrahydrofuran der folgenden Formel: 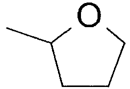
mit einem Säurehalogenid der folgenden allgemeinen Formel (12): 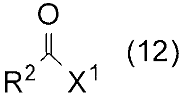
wobei R² eine Alkylgruppe mit 1 bis 9 Kohlenstoffatomen oder eine Phenylgruppe darstellt, und
X¹ wie oben definiert ist,
um eine 4-Halogenpentylacylat-Verbindung der folgenden allgemeinen Formel (13) zu erhalten: 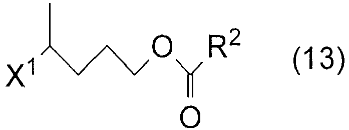
wobei X¹ und R² wie oben definiert sind,
Umsetzen der 4-Halogenpentylacylat-Verbindung (13) mit dem organometallischen Reagenz der allgemeinen Formel (14):
R³M³ (14)
wobei R³ eine Alkylgruppe mit 1 bis 14 Kohlenstoffatomen oder eine Ethinylgruppe darstellt, M³ Li, Na, K, Ag, MgZ⁴ oder CaZ⁴ darstellt und Z⁴ ein Halogenatom oder R³ darstellt, um eine 4-Halogen-1-pentanol-Verbindung der allgemeinen Formel (15) zu erhalten: 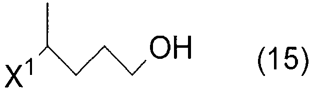
wobei X¹ wie oben definiert ist,
Unterziehen der 4-Halogen-1-pentanol-Verbindung (15) einer Alkoxymethylierung mit einer Halogenmethylalkylether-Verbindung der folgenden allgemeinen Formel (16): 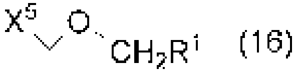
wobei R¹ wie oben definiert ist, und X⁵ ein Halogenatom darstellt,
um eine Halogenalkylalkoxymethylether-Verbindung der folgenden allgemeinen Formel (1A) zu bilden: 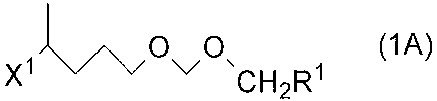
wobei X¹ und R¹ wie oben definiert sind.

5. Das Verfahren zur Herstellung von 4,6-Dimethyloctadecanol (7) gemäß Anspruch 2, wobei das Verfahren ferner die Schritte umfasst:
Öffnen des Tetrahydrofuranrings von 2-Methyltetrahydrofuran der folgenden Formel: 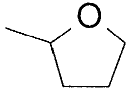
mit einem Säurehalogenid der folgenden allgemeinen Formel (12): 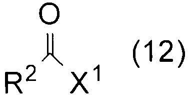
wobei R² eine Alkylgruppe mit 1 bis 9 Kohlenstoffatomen oder eine Phenylgruppe darstellt, und
X¹ wie oben definiert ist,
um eine 4-Halogenpentylacylatverbindung der folgenden allgemeinen Formel (13) zu erhalten: 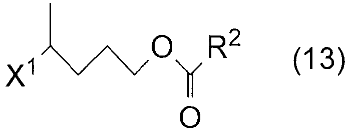
wobei X¹ und R² wie oben definiert sind,
Umsetzen der 4-Halogenpentylacylatverbindung (13) mit dem organometallischen Reagenz der allgemeinen Formel (14):
R³M³ (14)
wobei R³ eine Alkylgruppe mit 1 bis 14 Kohlenstoffatomen oder eine Ethinylgruppe darstellt, M³ Li, Na, K, Ag, MgZ⁴ oder CaZ⁴ darstellt und Z⁴ ein Halogenatom oder R³ darstellt,
um eine 4-Halogen-1-pentanol-Verbindung der allgemeinen Formel (15) zu erhalten: 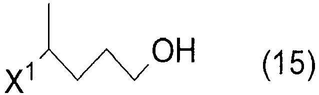
wobei X¹ wie oben definiert ist,
Unterziehen der 4-Halo-1-pentanol-Verbindung (15) einer Alkoxymethylierung mit einer Halogenmethylalkylether-Verbindung der folgenden allgemeinen Formel (16): 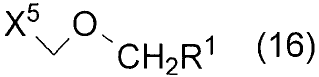
wobei R¹ wie oben definiert ist, und X⁵ ein Halogenatom darstellt,
um eine Halogenalkylalkoxymethylether-Verbindung der folgenden allgemeinen Formel (1A) zu bilden: 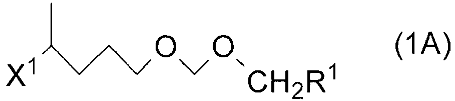
wobei X¹ und R¹ wie oben definiert sind.

6. Das Verfahren zur Herstellung von 13,15-Dimethylheptacosan (11) gemäß Anspruch 3, wobei das Verfahren ferner die Schritte umfasst:
Öffnen des Tetrahydrofuranrings von 2-Methyltetrahydrofuran der folgenden Formel: 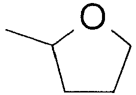
mit einem Säurehalogenid der folgenden allgemeinen Formel (12): 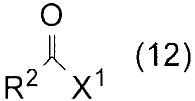
wobei R² eine Alkylgruppe mit 1 bis 9 Kohlenstoffatomen oder eine Phenylgruppe darstellt und X¹ wie oben definiert ist,
um eine 4-Halogenpentylacylat-Verbindung der folgenden allgemeinen Formel (13) zu erhalten: 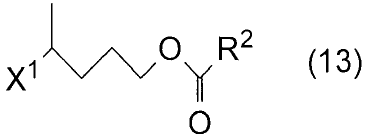
wobei X¹ und R² wie oben definiert sind,
Umsetzen der 4-Halogenpentylacylat-Verbindung (13) mit dem organometallischen Reagenz der allgemeinen Formel (14):
R³M³ (14)
wobei R³ eine Alkylgruppe mit 1 bis 14 Kohlenstoffatomen oder eine Ethinylgruppe darstellt,
M³ Li, Na, K, Ag, MgZ⁴ oder CaZ⁴ darstellt, und Z⁴ ein Halogenatom oder R³ darstellt, um eine 4-Halogen-1-pentanol-Verbindung der allgemeinen Formel (15) zu erhalten: 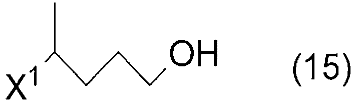
wobei X¹ wie oben definiert ist,
Unterziehen der 4-Halogen-1-pentanol-Verbindung (15) einer Alkoxymethylierung mit einer Halogenmethylalkyletherverbindung der folgenden allgemeinen Formel (16): 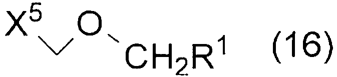
wobei R¹ wie oben definiert ist, und X⁵ ein Halogenatom darstellt,
um eine Halogenalkylalkoxymethylether-Verbindung der folgenden allgemeinen Formel (1A) zu bilden: 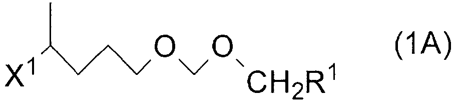
wobei X¹ und X² wie oben definiert sind.

7. Ein Verfahren zur Herstellung von 13,15-Dimethylheptacosan der folgenden allgemeinen Formel (11): 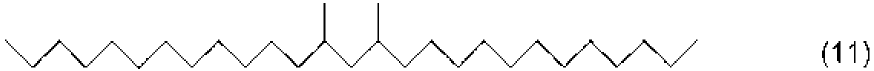 wobei das Verfahren die Schritte umfasst:
Umwandeln einer Halogenalkylalkoxymethylether-Verbindung der folgenden allgemeinen Formel (1B): 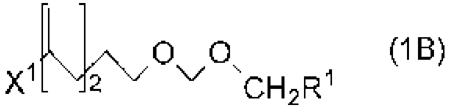
wobei X¹ ein Halogenatom darstellt und R¹ ein Wasserstoffatom, eine n-Alkylgruppe mit 1 bis 9 Kohlenstoffatomen oder eine Phenylgruppe darstellt,
in ein nukleophiles Reagenz, 6-Alkoxymethoxy-1,3-dimethylhexyl, der folgenden allgemeinen Formel (2B): 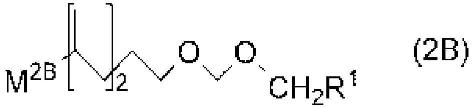
wobei M^{2B} Li, MgZ^{2B}, CuZ^{2B} oder CuLiZ^{2B} darstellt, Z^{2B} ein Halogenatom oder eine 6-Alkoxymethoxy-1,3-dimethylhexylgruppe darstellt, und R¹ wie oben definiert ist, anschließendes Unterziehen des nukleophilen Reagenz, 6-Alkoxymethoxy-1,3-dimethylhexyl (2B), einer Kupplungsreaktion mit einer 1-Halogendodecanverbindung der folgenden allgemeinen Formel (5):
X²(CH₂)₁₁CH₃ (5)
wobei X² ein Halogenatom darstellt,
um eine 4,6-Dimethyloctadecylalkoxymethylether-Verbindung der folgenden allgemeinen Formel (6) zu erhalten: 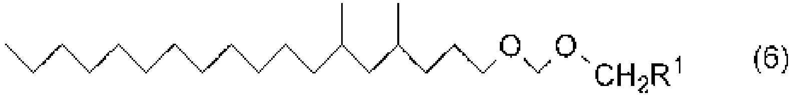
wobei R¹ wie oben definiert ist,
Unterziehen der 4,6-Dimethyloctadecylalkoxymethylether-Verbindung (6) einer Dealkoxymethylierungsreaktion, um 4,6-Dimethyloctadecanol der folgenden Formel (7) zu erhalten: 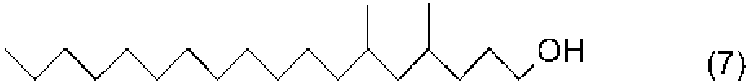
Unterziehen des 4,6-Dimethyloctadecanols (7) einer Halogenierungsreaktion, um eine 1-Halogen-4,6-dimethyloctadecan-Verbindung der folgenden allgemeinen Formel (8) zu erhalten: 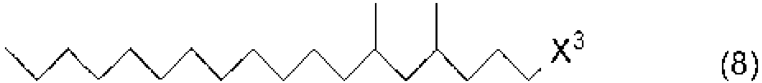
wobei X³ ein Halogenatom darstellt,
Umwandeln der 1-Halo-4,6-dimethyloctadecan-Verbindung (8) in ein nukleophiles Reagenz, 4,6-Dimethyloctadecyl, der folgenden allgemeinen Formel (9): 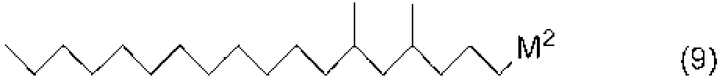 wobei M² Li, MgZ², CuZ² oder CuLiZ² darstellt, und Z² ein Halogenatom oder eine 4,6-Dimethyloctadecyl Gruppe darstellt,
und anschließendes Unterziehen des nukleophilen Reagenz, 4,6-Dimethyloctadecyl (9), einer Kupplungsreaktion mit einer 1-Halogennonanverbindung der folgenden allgemeinen Formel (10):
X⁴(CH₂)₈CH₃ (10)
wobei X⁴ ein Halogenatom darstellt,
um das vorgenannte 13,15-Dimethylheptacosan (11) zu bilden.

8. Eine Halogenalkylalkoxymethylether-Verbindung der folgenden allgemeinen Formel (1): 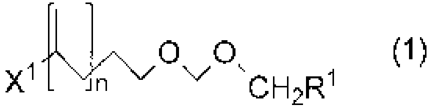 wobei X¹ ein Halogenatom darstellt, R¹ ein Wasserstoffatom, eine n-Alkylgruppe mit 1 bis 9 Kohlenstoffatomen oder eine Phenylgruppe darstellt und *n* 1 oder 2 darstellt.

9. Die Halogenalkylalkoxymethylether-Verbindung (1) gemäß Anspruch 8, wobei *n* gleich 1 ist, die durch die folgende allgemeine Formel (1A) dargestellt wird: 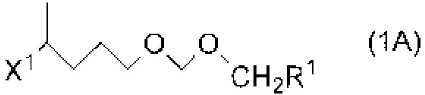 wobei X¹ ein Halogenatom darstellt und R¹ ein Wasserstoffatom, eine n-Alkylgruppe mit 1 bis 9 Kohlenstoffatomen oder eine Phenylgruppe darstellt.

10. Die Halogenalkylalkoxymethylether-Verbindung (1) gemäß Anspruch 8, wobei n gleich 2 ist, die durch die folgende allgemeine Formel (1B) dargestellt wird: 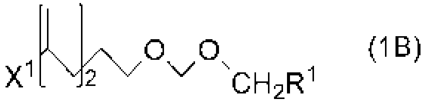 wobei X¹ ein Halogenatom darstellt und R¹ ein Wasserstoffatom, eine n-Alkylgruppe mit 1 bis 9 Kohlenstoffatomen oder eine Phenylgruppe darstellt.

## Revendications

1. Procédé de préparation d'un composé haloalkyl alcoxyméthyl éther de formule générale (1B) suivante : 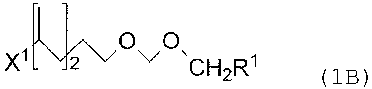
dans laquelle X¹ représente un atome d'halogène, et R¹ représente un atome d'hydrogène, un groupe n-alkyle ayant 1 à 9 atomes de carbone, ou un groupe phényle, le procédé comprenant les étapes de conversion d'un composé haloalkyl alcoxyméthyl éther de formule générale (1A) suivante : 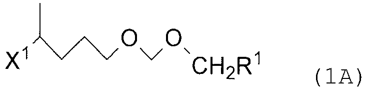
dans laquelle X¹ et R¹ sont tels que définis ci-dessus, en un réactif nucléophile, 4-alcoxyméthoxy-1-méthylbutyle, de formule générale (2A) suivante : 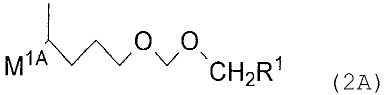
dans laquelle M^{1A} représente Li, MgZ^{1A}, CuZ^{1A}ou CuLiZ^{1A}, Z^{1A} représente un atome d'halogène ou un groupe 4-alcoxyméthoxy-1-méthylbutyle, et R¹ est tel que défini ci-dessus, de soumission ultérieure du réactif nucléophile, 4-alcoxyméthoxy-1-méthylbutyle (2A), à une réaction d'addition nucléophile avec de l'oxyde de propylène de formule (3) suivante : 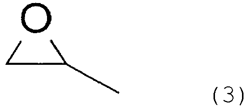
pour obtenir un composé 6-hydroxy-4-méthylheptyl alcoxyméthyl éther de formule générale (4) suivante : 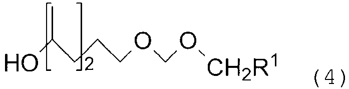
dans laquelle R¹ est tel que défini ci-dessus, et de soumission du composé 6-hydroxy-4-méthylheptyl alcoxyméthyl éther (4) à une réaction d'halogénation pour former le composé haloalkyl alcoxyméthyl éther (1B) susmentionné.

2. Procédé de préparation de 4,6-diméthyloctadécanol de formule (7) suivante : 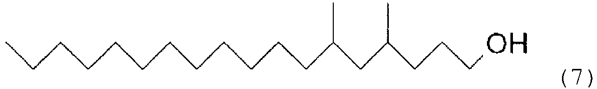
le procédé comprenant les étapes du procédé de préparation du composé haloalkyl alcoxyméthyl éther (1B) susmentionné selon la revendication 1,
de conversion du composé haloalkyl alcoxyméthyl éther (1B) en un réactif nucléophile, 6-alcoxyméthoxy-1,3-diméthylhexyle, de formule générale (2B) suivante : 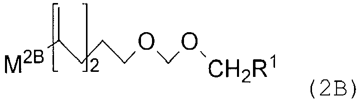
dans laquelle M^{2B} représente Li, MgZ^{2B}, CuZ^{2B}ou CuLiZ^{2B}, Z^{2B} représente un atome d'halogène ou un groupe 6-alcoxyméthoxy-1,3-diméthylhexyle, et R¹ est tel que défini ci-dessus, et de soumission ultérieure du réactif nucléophile, 6-alcoxyméthoxy-1,3-diméthylhexyle (2B), à une réaction de couplage avec un composé 1-halododécane de formule générale (5) suivante :
X²(CH₂)₁₁CH₃ (5)
dans laquelle X² représente un atome d'halogène, pour obtenir un composé 4,6-diméthyloctadécyl alcoxyméthyl éther de formule générale (6) suivante : 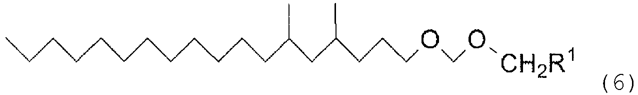
dans laquelle R¹ est tel que défini ci-dessus, et de soumission du composé 4,6-diméthyloctadécyl alcoxyméthyl éther (6) à une réaction de déalcoxyméthylation pour former le 4,6-diméthyloctadécanol (7) susmentionné.

3. Procédé de préparation de 13,15-diméthylheptacosane de formule générale (11) suivante : 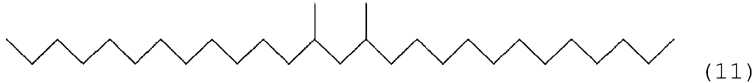
le procédé comprenant les étapes du procédé de préparation du composé haloalkyl alcoxyméthyl éther (1B) susmentionné selon la revendication 1, de conversion du composé haloalkyl alcoxyméthyl éther (1B) en un réactif nucléophile, 6-alcoxyméthoxy-1,3-diméthylhexyle, de formule générale (2B) suivante : 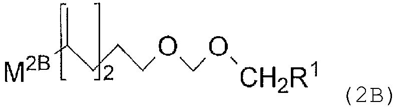
dans laquelle M^{2B} représente Li, MgZ^{2B}, CuZ^{2B} ou CuLiZ^{2B}, Z^{2B} représente un atome d'halogène ou un groupe 6-alcoxyméthoxy-1,3-diméthylhexyle, et R¹ est tel que défini ci-dessus, et de soumission ultérieure du réactif nucléophile, 6-alcoxyméthoxy-1,3-diméthylhexyle (2B), à une réaction de couplage avec un composé 1-halododécane de formule générale (5) suivante :
X²(CH₂)₁₁CH₃ (5)
dans laquelle X² représente un atome d'halogène, pour obtenir un composé 4,6-diméthyloctadécyl alcoxyméthyl éther de formule générale (6) suivante : 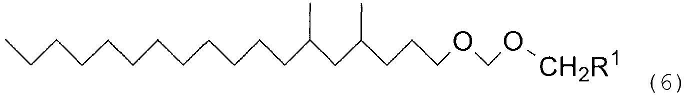
dans laquelle R¹ est tel que défini ci-dessus, de soumission du composé 4,6-diméthyloctadécyl alcoxyméthyl éther (6) à une réaction de déalcoxyméthylation pour obtenir du 4,6-diméthyloctadécanol de formule (7) suivante : 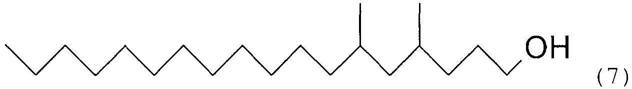
de soumission du 4,6-diméthyloctadécanol (7) à une réaction d'halogénation pour obtenir un composé 1-halo-4,6-diméthyloctadécane de formule générale (8) suivante : 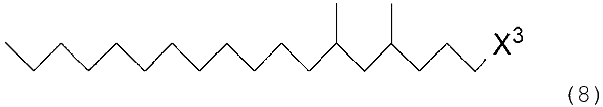
dans laquelle X³ représente un atome d'halogène, de conversion du composé 1-halo-4,6-diméthyloctadécane (8) en un réactif nucléophile, 4,6-diméthyloctadécyle, de formule générale (9) suivante : 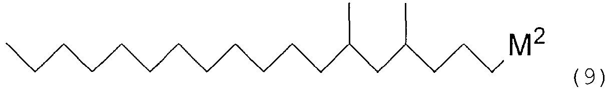
dans laquelle M² représente Li, MgZ², CuZ², ou CuLiZ², et Z² représente un atome d'halogène ou un groupe 4,6-diméthyloctadécyle, et de soumission ultérieure du réactif nucléophile, 4,6-diméthyloctadécyle (9), à une réaction de couplage avec un composé 1-halononane de formule générale (10) suivante :
X⁴(CH₂)₈CH₃ (10)
dans laquelle X⁴ représente un atome d'halogène, pour former le 13,15-diméthylheptacosane (11) susmentionné.

4. Procédé de préparation d'un composé haloalkyl alcoxyméthyl éther (1B) selon la revendication 1, le procédé comprenant en outre les étapes d'ouverture du cycle tétrahydrofurane de 2-méthyltétrahydrofurane de formule suivante : 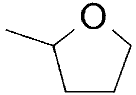
avec un halogénure d'acide de formule générale (12) suivante : 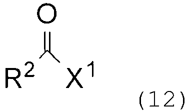
dans laquelle R² représente un groupe alkyle ayant 1 à 9 atomes de carbone ou un groupe phényle, et X¹ est tel que défini ci-dessus, pour obtenir un composé acylate de 4-halopentyle de formule générale (13) suivante : 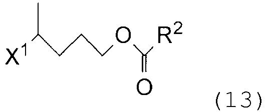
dans laquelle X¹ et R² sont tels que définis ci-dessus, de soumission du composé acylate de 4-halopentyle (13) au réactif organométallique de formule générale (14) :
R³M³ (14)
dans laquelle R³ représente un groupe alkyle ayant 1 à 14 atomes de carbone ou un groupe éthynyle, M³ représente Li, Na, K, Ag, MgZ⁴, ou CaZ⁴, et Z⁴ représente un atome d'halogène ou R³, pour obtenir un composé 4-halo-1-pentanol de formule générale (15) : 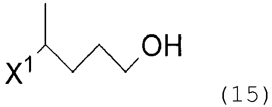
dans laquelle X¹ est tel que défini ci-dessus, de soumission du composé 4-halo-1-pentanol (15) à une alcoxyméthylation avec un composé halométhyl alkyl éther de formule générale (16) suivante : 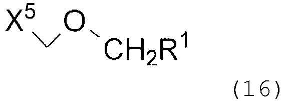
dans laquelle R¹ est tel que défini ci-dessus, et X⁵ représente un atome d'halogène, pour former un composé haloalkyl alcoxyméthyl éther de formule générale (1A) suivante : 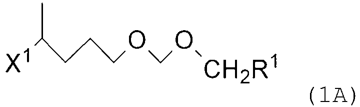
dans laquelle X¹ et R¹ sont tels que définis ci-dessus.

5. Procédé de préparation de 4,6-diméthyloctadécanol (7) selon la revendication 2, le procédé comprenant en outre les étapes d'ouverture du cycle tétrahydrofurane de 2-méthyltétrahydrofurane de formule suivante : 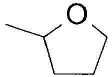
avec un halogénure d'acide de formule générale (12) suivante : 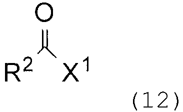
dans laquelle R² représente un groupe alkyle ayant 1 à 9 atomes de carbone ou un groupe phényle, et X¹ est tel que défini ci-dessus, pour obtenir un composé acylate de 4-halopentyle de formule générale (13) suivante : 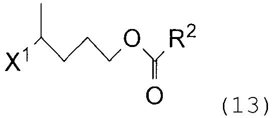
dans laquelle X¹ et R² sont tels que définis ci-dessus, de soumission du composé acylate de 4-halopentyle (13) au réactif organométallique de formule générale (14) :
R³M³ (14)
dans laquelle R³ représente un groupe alkyle ayant 1 à 14 atomes de carbone ou un groupe éthynyle, M³ représente Li, Na, K, Ag, MgZ⁴, ou CaZ⁴, et Z⁴ représente un atome d'halogène ou R³, pour obtenir un composé 4-halo-1-pentanol de formule générale (15) : 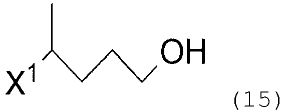
dans laquelle X¹ est tel que défini ci-dessus, de soumission du composé 4-halo-1-pentanol (15) à une alcoxyméthylation avec un composé halométhyl alkyl éther de formule générale (16) suivante : 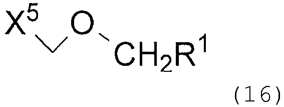
dans laquelle R¹ est tel que défini ci-dessus, et X⁵ représente un atome d'halogène, pour former un composé haloalkyl alcoxyméthyl éther de formule générale (1A) suivante : 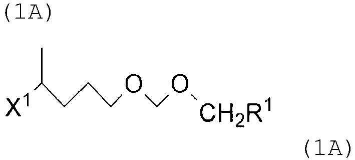
dans laquelle X¹ et R¹ sont tels que définis ci-dessus.

6. Procédé de préparation de 13,15-diméthylheptacosane (11) selon la revendication 3, le procédé comprenant en outre les étapes d'ouverture du cycle tétrahydrofurane de 2-méthyltétrahydrofurane de formule suivante : 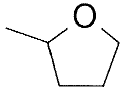
avec un halogénure d'acide de formule générale (12) suivante : 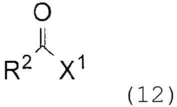
dans laquelle R² représente un groupe alkyle ayant 1 à 9 atomes de carbone ou un groupe phényle, et X¹ est tel que défini ci-dessus, pour obtenir un composé acylate de 4-halopentyle de formule générale (13) suivante : 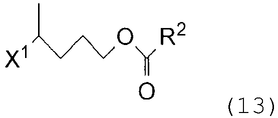
dans laquelle X¹ et R² sont tels que définis ci-dessus, de soumission du composé acylate de 4-halopentyle (13) au réactif organométallique de formule générale (14) :
R³M³ (14)
dans laquelle R³ représente un groupe alkyle ayant 1 à 14 atomes de carbone ou un groupe éthynyle, M³ représente Li, Na, K, Ag, MgZ⁴, ou CaZ⁴, et Z⁴ représente un atome d'halogène ou R³, pour obtenir un composé 4-halo-1-pentanol de formule générale (15) : 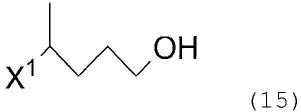
dans laquelle X¹ est tel que défini ci-dessus, de soumission du composé 4-halo-1-pentanol (15) à une alcoxyméthylation avec un composé halométhyl alkyl éther de formule générale (16) suivante : 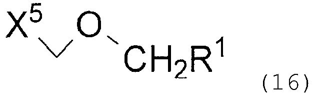
dans laquelle R¹ est tel que défini ci-dessus, et X⁵ représente un atome d'halogène, pour former un composé haloalkyl alcoxyméthyl éther de formule générale (1A) suivante : 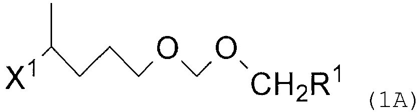
dans laquelle X¹ et R¹ sont tels que définis ci-dessus.

7. Procédé de préparation de 13,15-diméthylheptacosane de formule générale (11) suivante : 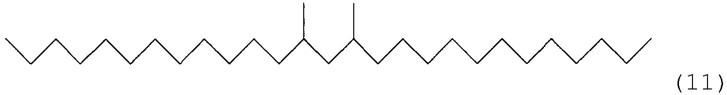
le procédé comprenant les étapes de conversion d'un composé haloalkyl alcoxyméthyl éther de formule générale (1B) suivante : 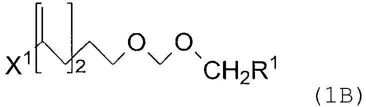
dans laquelle X¹ représente un atome d'halogène, et R¹ représente un atome d'hydrogène, un groupe n-alkyle ayant 1 à 9 atomes de carbone, ou un groupe phényle, en un réactif nucléophile, 6-alcoxyméthoxy-1,3-diméthylhexyle, de formule générale (2B) suivante : 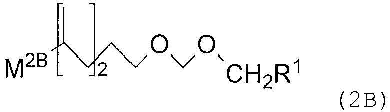
dans laquelle M^{2B} représente Li, MgZ^{2B}, CuZ^{2B}ou CuLiZ^{2B}, Z^{2B} représente un atome d'halogène ou un groupe 6-alcoxyméthoxy-1,3-diméthylhexyle, et R¹ est tel que défini ci-dessus, de soumission ultérieure du réactif nucléophile, 6-alcoxyméthoxy-1,3-diméthylhexyle (2B), à une réaction de couplage avec un composé 1-halododécane de formule générale (5) suivante :
X²(CH₂)₁₁CH₃ (5)
dans laquelle X² représente un atome d'halogène, pour obtenir un composé 4,6-diméthyloctadécyl alcoxyméthyl éther de formule générale (6) suivante : 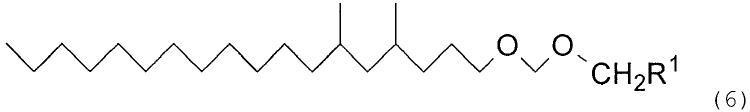
dans laquelle R¹ est tel que défini ci-dessus, de soumission du composé 4,6-diméthyloctadécyl alcoxyméthyl éther (6) à une réaction de déalcoxyméthylation pour obtenir du 4,6-diméthyloctadécanol de formule (7) suivante : 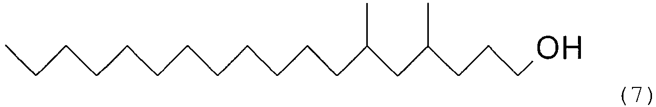
de soumission du 4,6-diméthyloctadécanol (7) à une réaction d'halogénation pour obtenir un composé 1-halo-4,6-diméthyloctadécane de formule générale (8) suivante : 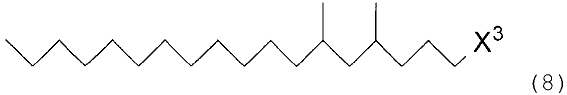
dans laquelle X³ représente un atome d'halogène, de conversion du composé 1-halo-4,6-diméthyloctadécane (8) en un réactif nucléophile, 4,6-diméthyloctadécyle, de formule générale (9) suivante : 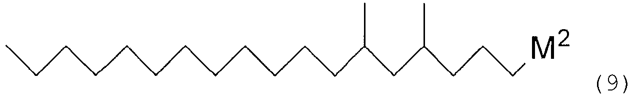
dans laquelle M² représente Li, MgZ², CuZ², ou CuLiZ², et Z² représente un atome d'halogène ou un groupe 4,6-diméthyloctadécyle,
et de soumission ultérieure du réactif nucléophile, 4,6-diméthyloctadécyle (9), à une réaction de couplage avec un composé 1-halononane de formule générale (10) suivante :
X⁴(CH₂)₈CH³ (10)
dans laquelle X⁴ représente un atome d'halogène, pour former le 13,15-diméthylheptacosane (11) susmentionné.

8. Composé haloalkyl alcoxyméthyl éther de formule générale (1) suivante : 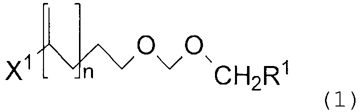 dans laquelle X¹ représente un atome d'halogène, R¹ représente un atome d'hydrogène, un groupe n-alkyle ayant 1 à 9 atomes de carbone, ou un groupe phényle, et n représente 1 ou 2.

9. Composé haloalkyl alcoxyméthyl éther (1) selon la revendication 8, dans lequel n est 1, qui est représenté par la formule générale (1A) suivante : 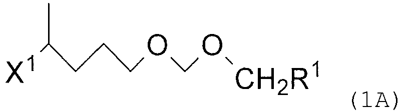 dans laquelle X¹ représente un atome d'halogène, et R¹ représente un atome d'hydrogène, un groupe n-alkyle ayant 1 à 9 atomes de carbone, ou un groupe phényle.

10. Composé haloalkyl alcoxyméthyl éther (1) selon la revendication 8, dans lequel n est 2, qui est représenté par la formule générale (1B) suivante : 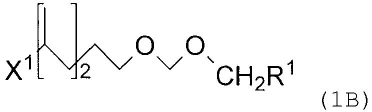 dans laquelle X¹ représente un atome d'halogène, et R¹ représente un atome d'hydrogène, un groupe n-alkyle ayant 1 à 9 atomes de carbone, ou un groupe phényle.
